(19) **Europäisches Patentamt**
**European Patent Office**
**Office européen des brevets**

(11) **EP 3 372 154 B1**

(12) **EUROPEAN PATENT SPECIFICATION**

(45) Date of publication and mention
of the grant of the patent:
**04.12.2019 Bulletin 2019/49**

(51) Int Cl.:
*A61B 5/0245* (2006.01)    *A61B 5/00* (2006.01)
*A61B 5/024* (2006.01)    *G16H 20/60* (2018.01)

(21) Application number: **15907809.6**

(22) Date of filing: **05.11.2015**

(86) International application number:
**PCT/JP2015/081192**

(87) International publication number:
**WO 2017/077623 (11.05.2017 Gazette 2017/19)**

(54) **DRINKING DETECTION BY HEART RATE ANALYSIS**

ERKENNEN VON TRINKEREIGNISSEN MITTELS HERZRATENANALYSE

DÉTECTION DE LA CONSOMMATION DE BOISSONS PAR ANALYSE DE LA FRÉQUENCE CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.09.2018 Bulletin 2018/37**

(73) Proprietor: **Fujitsu Limited**
**Kawasaki-shi, Kanagawa 211-8588 (JP)**

(72) Inventors:
• **WASHIZAWA, Shiho**
**Kawasaki-shi**
**Kanagawa 211-8588 (JP)**
• **MAEDA, Kazuho**
**Kawasaki-shi**
**Kanagawa 211-8588 (JP)**
• **UCHIDA, Daisuke**
**Kawasaki-shi**
**Kanagawa 211-8588 (JP)**
• **MORI, Tatsuya**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**Lincoln House, 5th Floor**
**300 High Holborn**
**London WC1V 7JH (GB)**

(56) References cited:
**JP-A- H10 504 739    JP-A- 2000 245 713**
**JP-A- 2000 245 713    JP-A- 2003 173 375**
**JP-A- 2007 048 180    JP-A- 2010 158 267**
**US-A1- 2008 300 641**

EP 3 372 154 B1

**Description**

FIELD

The present case relates to an information processing device, an information processing method and an information processing program.

BACKGROUND

A technique for detecting a drink intake has been desired. For example, disclosed is a technique for determining a drink intake by detecting a motion of an arm during the drink intake (see Patent Document 1, for example).

PRIOR ART DOCUMENT

PATENT DOCUMENT

Patent Document 1: Japanese Patent Application Publication No. 2014-79506
JP2000245713 (A) discloses a wristwatch type sensor part provided with a skin temperature sensor and a pulse sensor and a belt installing type sensor provided with an acceleration sensor that are connected to a personal computer connected with a display. A behaviour classification such as sleep, eating and drinking, stress, physical exercises and a rest is judged by a behaviour classification judging part on the basis of an analytical result in a skin temperature analysing part, a pulse analysing part and an acceleration analysing part for inputting output signal of the respective sensors. Judged behaviour pattern information is displayed on a display, and sleeping time and physical exercise time are evaluated by an improving behaviour detecting part referring to a pre-set rule.
US2008300641 (A1) discloses a cardiac sensor adapted to generate cardiac information descriptive of cardiac functioning of a patient, an activity sensor adapted to generate activity information indicating physical activity of the patient, a processing element adapted to detect a cardiac anomaly based on the cardiac information, an information association element adapted to generate associated cardiac/activity information during the cardiac anomaly, and a data storage apparatus adapted to store the associated cardiac/activity information. A method embodiment includes generating cardiac information descriptive of cardiac functioning of a patient, detecting a cardiac anomaly based on the cardiac information, generating activity information descriptive of physical activity of the patient during the cardiac anomaly, associating the cardiac information and the activity information, during the cardiac anomaly, to generate associated cardiac/activity information, and storing the associated cardiac/activity information.

DISCLOSURE OF THE INVENTION

PROBLEMS TO BE SOLVED BY THE INVENTION

However, there are various motions of an arm during a drink intake. It is therefore difficult to accurately detect an action of the drink intake. For example, the motion of the arm assumed in the above-mentioned invention is only a part of motions during the drink intake. The drink intake may not be necessarily detected during other motions during the drink intake. There are various motions of an arm in a daily life. Therefore, a false detection may occur.
According to an aspect of the present case, provided is an information processing device, an information processing method, and an information processing program capable of detecting a drink intake under various conditions, independently of a specific action.

MEANS FOR SOLVING THE PROBLEMS

The present invention is defined by the appended independent claims, to which reference should now be made. Specific embodiments are defined in the dependent claims..

EFFECTS OF THE INVENTION

It is possible to detect a drink intake under various conditions, independently of a specific action.

BRIEF DESCRIPTION OF DRAWINGS

FIG. 1 is a block diagram of a hardware configuration of an information processing device in accordance with a first embodiment;

FIG. 2A is a block diagram of functions implemented by execution of an information processing program, and FIG. 2B is a functional block diagram of functions of a heartbeat fluctuation calculation unit;

FIG. 3 illustrates an example of a flowchart of a drink intake detection process;

FIG. 4 illustrates heart rate fluctuation caused by drink intake;

FIG. 5 illustrates an example of a flowchart of a calculation process of a feature;

FIG. 6 illustrates a calculation of a speed feature;

FIG. 7A illustrates an example of a flowchart of a calculation process of a speed feature i to a speed feature iii, FIG. 7B illustrates an example of a flowchart of a calculation process of a difference feature iv to a difference feature vi, FIG. 7C illustrates an example of a flowchart of a calculation process of an intersection feature vii;

FIG. 8 illustrates a calculation of a difference feature;

FIG. 9 illustrates a calculation of an intersection feature;

FIG. 10 illustrates a calculation of a baseline feature;

FIG. 11A illustrates an example of a flowchart of a calculation process of a baseline feature, FIG. 11B illustrates an example of a flowchart of a calculation process of a fluctuation feature, FIG. 11C illustrates an example of a flowchart of a calculation process of an amplitude feature, FIG. 11D illustrates an example of a flowchart of a calculation process of an area feature;

FIG. 12 illustrates a calculation of a fluctuation feature;

FIG. 13 illustrates a calculation of a heartbeat feature;

FIG. 14 illustrates a calculation of an area feature;

FIG. 15 illustrates another example of approximation;

FIG. 16 illustrates an example of a flowchart of making a model stored in a database;

FIG. 17 illustrates an example of a flowchart of determining drink intake or drink non-intake;

FIG. 18 illustrates a Mahalanobis distance;

FIG. 19 illustrates an example of a flowchart of a calculation of amount of taken drink;

FIG. 20 illustrates a block diagram of a hardware structure of an information processing device in accordance with a second embodiment;

FIG. 21 illustrates an example of a flowchart of a drink intake detection process;

FIG. 22A illustrates a functional block diagram of an information processing device in accordance with a third embodiment, FIG. 22B illustrates a functional block diagram of each function of a heartbeat fluctuation unit;

FIG. 23 illustrates an example of a flowchart of a drink intake detection process;

FIG. 24A illustrates a heart rate fluctuation signal s(t) with respect to time of a drink intake, FIG. 24B illustrates a heart rate fluctuation signal x(t) with respect to time of a drink intake detection process; and

FIG. 25A and FIG. 25B illustrate another device configuration

Of an information processing device.

DESCRIPTION OF EMBODIMENTS

Hereinafter, a description will be given of embodiments with reference to drawings.

(First Embodiment)

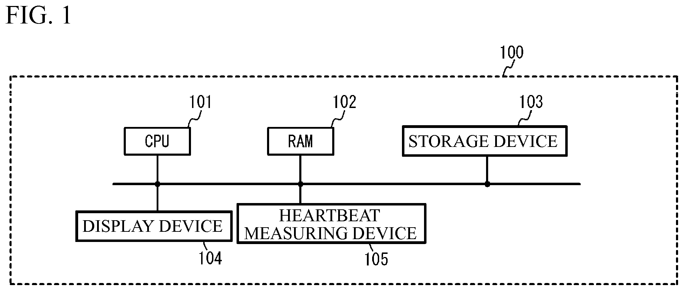

FIG. 1 is a block diagram of a hardware configuration of an information processing device 100 in accordance with a first embodiment. As illustrated in FIG. 1, the information processing device 100 includes a CPU 101, a RAM 102, a storage device 103, a display device 104, a heartbeat measuring device 105 and the like. These devices are interconnected through a bus or the like.

The CPU (Central Processing Unit) 101 is a Central Processing Unit. The CPU 101 includes one or more cores. The RAM (Random Access Memory) 102 is a volatile memory that temporarily stores programs executed by the CPU 101 and data processed by the CPU 101.

The storage device 103 is a nonvolatile storage device. For example, a ROM (Read Only Memory), a solid state drive (SSD) such as a flash memory, or a hard disk driven by a hard disk drive can be used as the storage device 103. The information processing program of the present embodiment is stored in the storage device 103. The display device 104 is a liquid crystal display, an electroluminescence panel, or the like, and displays the result of a drink intake detection process described later.

The heartbeat measuring device 105 is a device that measures the heartbeat (pulse) of an object such as a person or an animal taking a drink. The heartbeat measuring device 105 is not particularly limited as long as it can measure heartbeat (pulse). For example, the heartbeat measuring device 105 may be an electrocardiograph or a pulsation sensor. The drink not particularly limited as long as it is a liquid drink. However, when an alcohol is taken, there may be a case where a heart rate may increase because of absorption or degradation of the alcohol after taking the alcohol. It is therefore preferable that a non-alcohol drink is used as the drink in the embodiment.

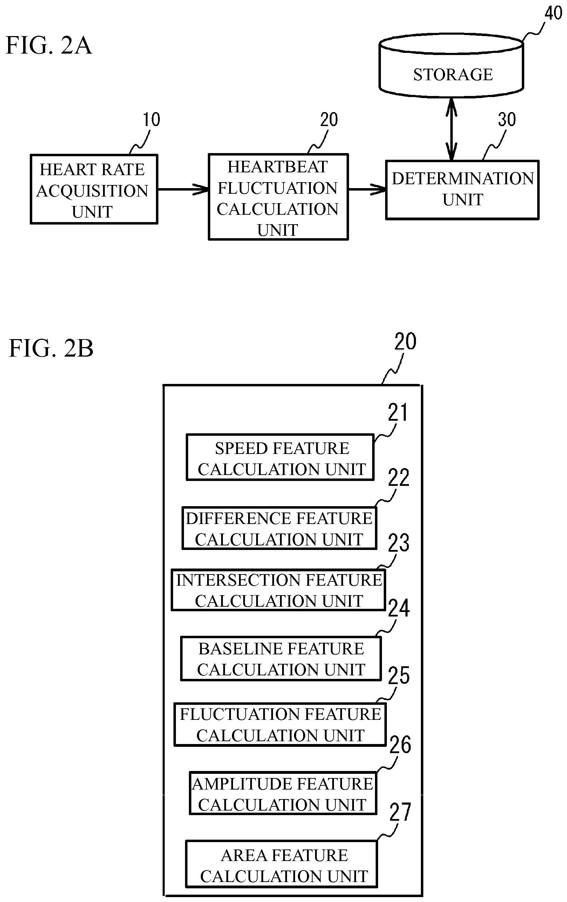

The information processing program stored in the storage device 103 is deployed in the RAM 102 in an executable manner. The CPU 101 executes the information processing program deployed in the RAM 102. This causes the information processing device 100 to execute each process. FIG. 2A is a block diagram of functions implemented by the execution of the information processing program. The execution of the information processing program implements a heart rate acquisition unit 10, a heartbeat fluctuation calculation unit 20, a determination unit 30, a storage 40 and so on. In the following, the storage means a database.

FIG. 2B is a functional block diagram of functions of the heartbeat fluctuation calculation unit 20. As illustrated in FIG. 2B, the heartbeat fluctuation calculation unit 20 functions as a speed feature calculation unit 21, a difference feature calculation unit 22, an intersection feature calculation unit 23, a baseline feature calculation unit 24, a fluctuation feature calculation unit 25, an amplitude feature calculation unit 26, an area feature calculation unit 27 and so on.

(Drink intake detection process)

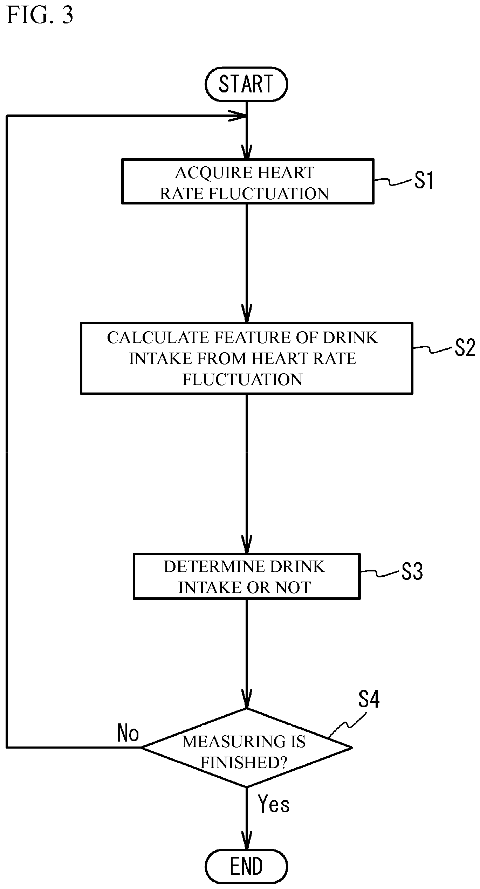

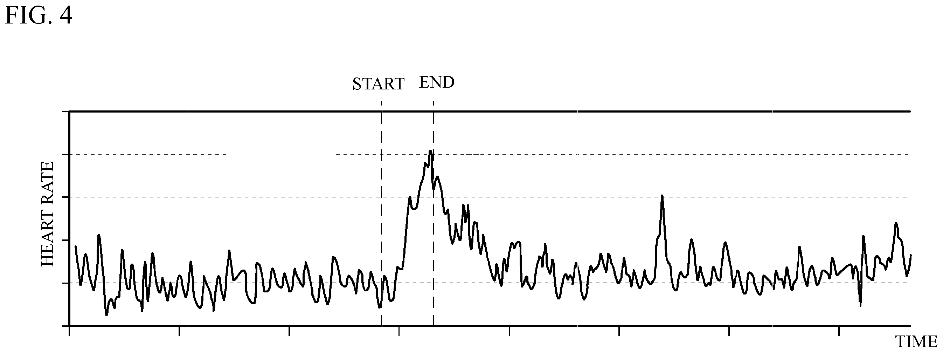

FIG. 3 illustrates an example of a flowchart of a drink intake detection process. As illustrated in FIG. 3, the heart rate acquisition unit 10 acquires heart rate fluctuation with respect to time by acquiring heartbeat from the heartbeat measuring device 105 (Step S1). FIG. 4 illustrates heart rate fluctuation with respect to time caused by a drink intake. In FIG. 4, a horizontal axis indicates an elapsed time, and a vertical axis indicates a heart rate. The heart rate is the number of beats per unit time, more specifically, the number of beats per minute. Hereinafter, unless otherwise specified, the heart rate means the number of beats per minute. The drink intake means that a drink is swallowed down one's throat once or more within a predetermined time range. When a new drink is swallowed after the predetermined time range, a second drink intake is performed. FIG. 4 illustrates heart rate fluctuation caused by a single drink intake.

As illustrated in FIG. 4, the heart rate fluctuation with respect to time caused by the drink intake has a tendency in which the heart rate rapidly increases at a start time of the drink intake and slowly decreases after an end of the drink intake.
The fluctuation has a tendency in which the heart rate increases at a constant speed at the start time of the drink intake and decreases at a variable speed after the end of the drink intake. The fluctuation has a tendency in which the heart rate decreases just after rapid increasing of the heat rate at the start time of the drink intake. The Fluctuation has a tendency in which a baseline (average value) of the heart rate after the drink intake is larger than a baseline of the heart rate before the drink intake. The fluctuation has a tendency in which a variation (dispersion) of the heart rate after the drink intake is larger than the variation of the heart rate before the drink intake. And so, it is possible to determine whether an object takes a drink or not, by storing these tendencies in a database in advance and comparing measured heart rate fluctuation of the object with these tendencies. The database includes a text file and a binary file.

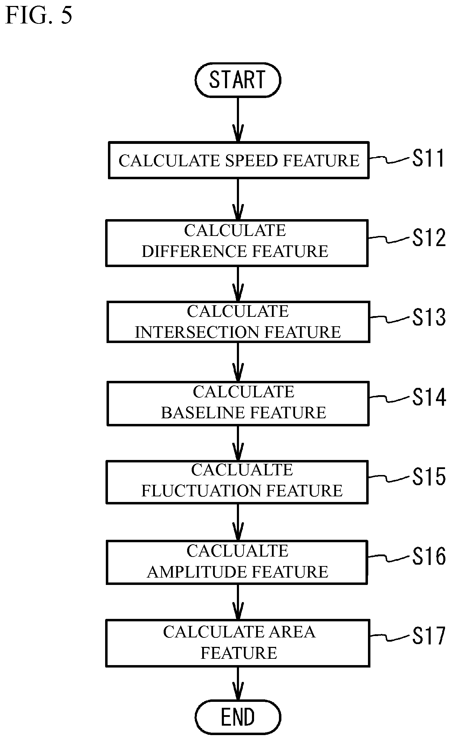

As illustrated in FIG. 3, the heartbeat fluctuation calculation unit 20 calculates a feature of the drink intake in the heart rate fluctuation acquired by the heart rate acquisition unit 10 (Step S2). FIG. 5 illustrates an example of a flowchart of a calculation process of a feature. The speed feature calculation unit 21 calculates a speed feature of the heart rate fluctuation because the heart rate fluctuation of the drink intake has a tendency in which the heart rate rapidly increases at a start time of the drink intake and slowly decreases after the drink intake (Step S11).

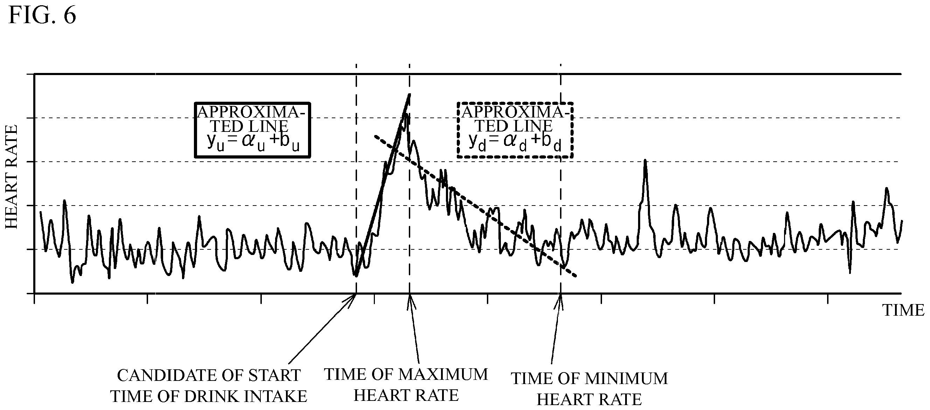

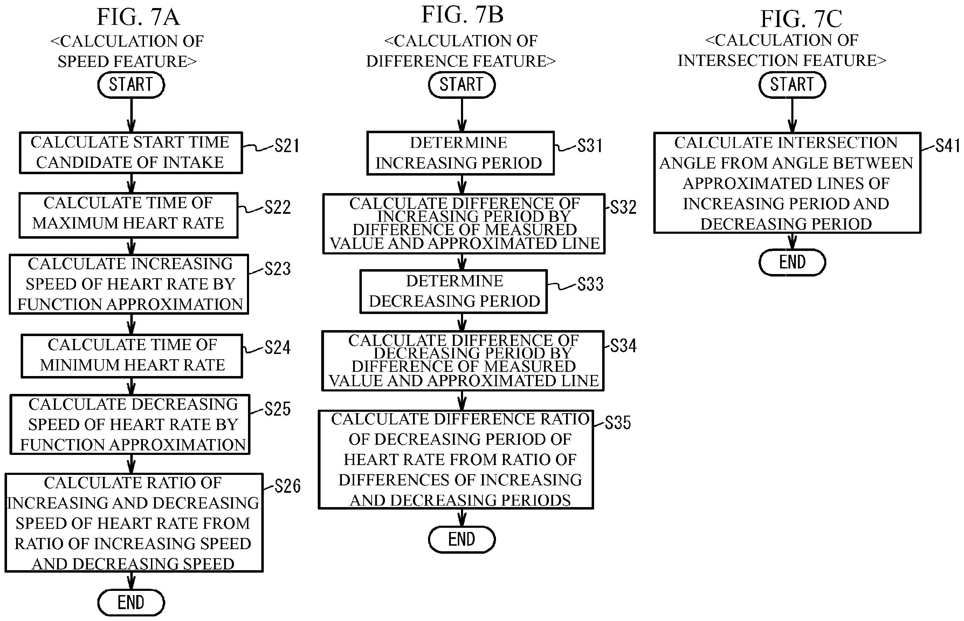

As a speed feature, the speed feature calculation unit 21 calculates a heart rate increasing speed (speed feature i) from a start time candidate of the drink intake, a heart rate decreasing speed (speed feature ii), and a ratio of the heart rate increasing speed and the heart rate decreasing speed (speed ratio of increasing and decreasing of the heart rate: speed feature iii). FIG. 6 illustrates examples of calculation of the speed feature i to the speed feature iii. FIG. 7A illustrates an example of a flowchart of a calculation process of the speed feature i to the speed feature iii.

As illustrated in FIG. 7A, the speed feature calculation unit 21 calculates a start time candidate of the drink intake (Step S21). For example, the start time candidate of the drink intake is a start time of increasing during an increasing period of the heart rate for a predetermined time (for example, 5 seconds) or more. The start time candidate of the drink intake may be a time point at which a rising of the heart rate is detected. Next, the speed feature calculation unit 21 calculates a time of a maximum heart rate (Step S22). For example, the maximum heart rate is a maximum value of the heart rate during the increasing period of the heart rate. The increasing period of the heart rate may be considered as a constant multiplication of period from time when an object holds a drink in his mouth to time when the object swallows the drink down his throat (for example, from time when the object holds 20 ml of the drink in his mouth to time when the object swallows the drink at a time (for example, 3 seconds)). For example, the multiplication of the time is a period in which water of 20 ml in 1 litter is continuously swallowed 50 times (for example, 150 seconds).

Next, the speed feature calculation unit 21 calculates the heart rate increasing speed by a function approximation (Step S23). For example, the speed feature calculation unit 21 calculates the heart rates from the start time candidate of the drink intake to the time of the maximum heart rate and calculates an inclination $a_u$ of an approximated line obtained by a function approximation of the calculated heart rates.

Next, the speed feature calculation unit 21 calculates time of a minimum heart rate (Step S24). For example, the minimum heart rate is a minimum value of heart rates in a heart rate decreasing period. The heart rate decreasing period may be considered as a period from time when a drink is swallowed to time when the liquid reaches a stomach (a period until the swallowed drink reaches the stomach (for example, 10 seconds)). Next, the speed feature calculation unit 21 calculates the heart rate decreasing speed by a function approximation (Step S24). For example, the speed feature calculation unit 21 calculates the heart rates from the time of the maximum heart rate to the time of the minimum heart rate and calculates an inclination $a_d$ of an approximated line obtained by a function approximation of the calculated heart rates, as the heart rate decreasing speed.

Next, the speed feature calculation unit 21 calculates a ratio of the heart rate increasing speed and the heart rate decreasing speed ($|a_u|/|a_d|$) as the speed ratio of increasing and decreasing of the heart rate (Step S26). A value indicating a difference between the increasing and the decreasing may be used instead of the speed ratio. And so, the speed feature calculation unit 21 may calculate a difference between the increasing speed and the decreasing speed ($|a_u| - |a_d|$) as the speed feature iii. The speed feature calculation unit 21 calculates at least one of the speed feature i to the speed feature iii.

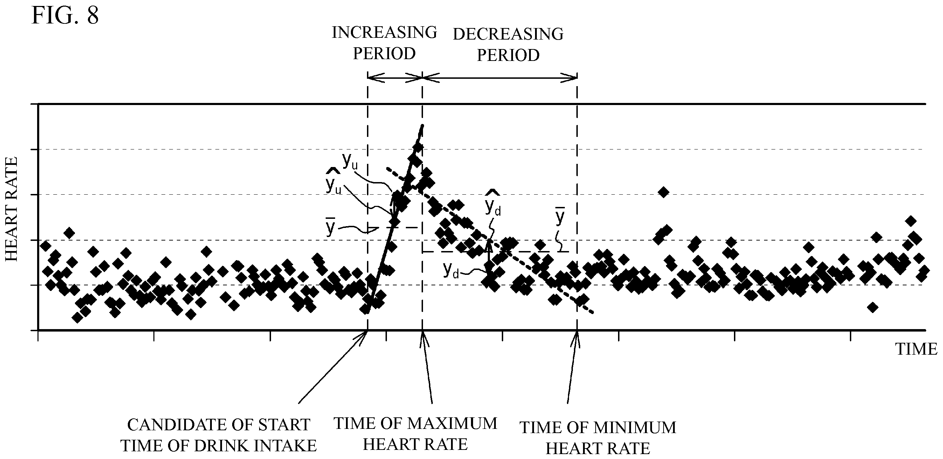

Next, the heart rate fluctuation of the drink intake with respect to time has the tendency in which the heart rate increases at a constant speed at the start time of the drink intake and decreases at a variable speed after the end of the drink intake. Therefore, as illustrated in FIG. 5, the difference feature calculation unit 22 calculates the difference feature of the heart rate fluctuation (Step S12). In concrete, as the difference feature, the difference feature calculation unit 22 calculates a difference between a measured value and the approximated line within the increasing period (difference within the increasing period: difference feature iv), a difference between a measured value and the approximated line of the decreasing period (difference within the decreasing period: difference feature v), and a ratio of the difference of the increasing period and the difference of the decreasing period (ratio of the difference between the increasing period and the decreasing period: difference feature vi). The difference may be called an error between the measured value and the approximated line. FIG. 7B illustrates an example of a flowchart of a calculation process of the difference feature iv to the difference feature vi. FIG. 8 illustrates a calculation of the difference feature iv to the difference feature vi.

As illustrated in FIG. 7B, the difference feature calculation unit 22 determines the heart rate increasing period (Step S31). For example, the increasing period is a period from the start time candidate of the drink intake to the time of the maximum heart rate. Next, the difference feature calculation unit 22 calculates the difference within the increasing period from the difference between the measured value and the approximated line (Step S32). For example, the difference within the increasing period is a value corresponding to a coefficient of determination $R_u$ of the measured value and the approximated line of the increasing period. The coefficient of determination $R_u$ may be expressed by the following formula (1).

[Formula 1]

$$R_u = \left(\sum_{i=1}^{M_u}(\hat{y}_i - \bar{y})^2\right) / \left(\sum_{i=1}^{M_u}(y_i - \bar{y})^2\right) \quad (1)$$

$y_i$ : measured value, $\bar{y}$ : average, $\hat{y}_i$ : predicted value
$M_u$ : number of measured data

Next, the difference feature calculation unit 22 determines the decreasing period (Step S33). For example, the decreasing period is a period from the time of the maximum heart rate to the time of the minimum heart rate. Next, the difference feature calculation unit 22 calculates the difference of the decreasing period from the difference between the measured value and the approximated line (Step S34). For example, the difference of the decreasing period is a value corresponding to a coefficient of determination $R_d$ of the measured value and the approximated line of the decreasing period. $R_d$ may be expressed by the following formula (2).

[Formula 2]

$$R_d = \left(\sum_{i=1}^{M_d}(\hat{y}_i - \bar{y})^2\right) / \left(\sum_{i=1}^{M_d}(y_i - \bar{y})^2\right) \quad (2)$$

$y_i$ : measured value, $\bar{y}$ : average, $\hat{y}_i$: predicted value
$M_d$ : number measured data

Next, as the difference ratio between the increasing period and the increasing period, the difference feature calculation unit 22 calculates a value corresponding to the ratio Ru/Rd of the difference of the increasing period and the difference of the decreasing period (Step S35). A value indicating a difference between the increasing and the decreasing may be used instead of the difference ratio. And so, the difference feature calculation unit 22 may calculate a difference between the difference of the increasing period and difference of the decreasing period ($|R_u| - |R_d|$) as the difference feature vi. The difference feature calculation unit 22 calculates at least one of the difference feature iv to the difference feature vi.

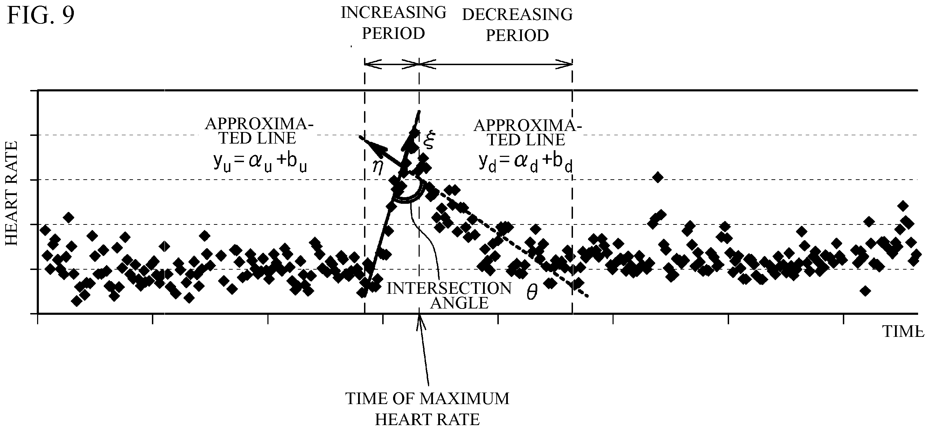

Next, the heart rate fluctuation of the drink intake has a tendency in which the heart rate decreases just after rapid increasing of the heat rate at the star time of the drink intake. Therefore, as illustrated in FIG. 5, the intersection feature calculation unit 23 calculates an intersection feature of the heart rate fluctuation (Step S13). In concrete, as the intersection feature, the intersection feature calculation unit 23 calculates an intersection angle of feature (intersection feature vii) indicating decreasing just after the time of the maximum heart rate. FIG. 9 illustrates a calculation of the intersection feature vii. FIG. 7C illustrates an example of a flowchart of a calculation process of the intersection feature vii. As illustrated in FIG. 7C, the intersection feature calculation unit 23 calculates the intersection angle $\theta$ as an intersection angle between the approximated line of the increasing period and the approximated line of the decreasing period (Step S41). The intersection angle $\theta$ may be calculated by the following formula (3) and the following formula (4).

[Formula 3]

$$\xi = \left( \frac{1}{\sqrt{1+a_u{}^2}}, \frac{a_u}{\sqrt{1+a_u{}^2}} \right)^T, \eta = \left( \frac{1}{\sqrt{1+a_d{}^2}}, \frac{a_d}{\sqrt{1+a_d{}^2}} \right) \quad (3)$$

[Formula 4]

$$\theta = \cos^{-1}\left( \frac{\langle \xi \mid \eta \rangle}{\|\xi\|\|\eta\|} \right) \quad (4)$$

Next, the heart rate fluctuation of the drink intake has the tendency in which a baseline of the heart rate after the drink intake is larger than a baseline of the heart rate before the drink intake. Therefore, as illustrated in FIG. 5, the baseline feature calculation unit 24 calculates a baseline feature of the heart rate fluctuation (Step S14). In concrete, as the baseline feature, the baseline feature calculation unit 24 calculates a baseline before the increasing period (baseline feature viii), a baseline feature after the decreasing period (baseline feature ix) and a ratio of the baseline before the increasing period and the baseline after the decreasing period (a baseline ratio between before the increasing and after the decreasing: baseline feature).

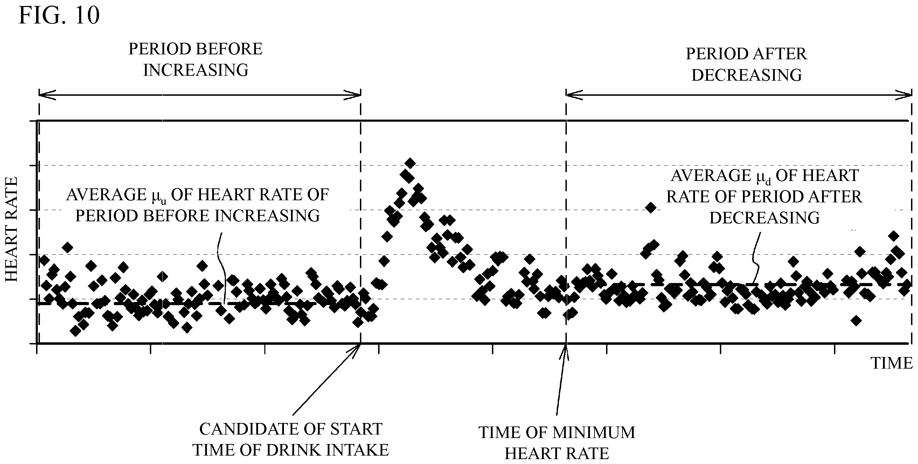

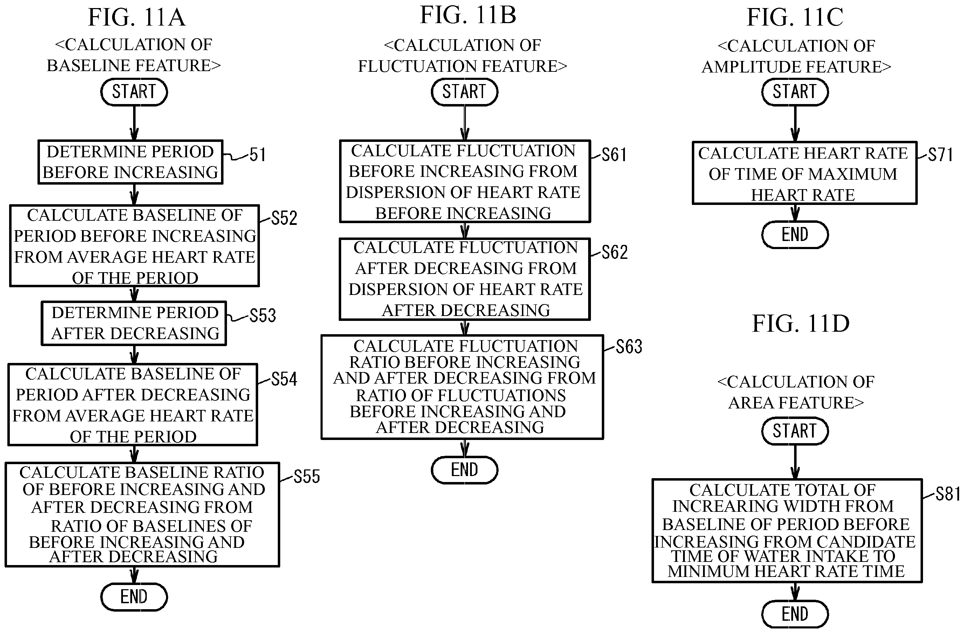

FIG. 10 illustrates a calculation of the baseline feature viii to the baseline feature ix. FIG. 11A illustrates an example of a flowchart of a calculation process of the baseline feature viii to the baseline feature ix. As illustrated in FIG. 11A, the baseline feature calculation unit 24 determines the period before the increasing (Step S51). For example, the period before the increasing is a period from a predetermined time before the start time candidate of the drink intake to the start time candidate of the drink intake. The predetermined time may be two minute or 30 minutes, for example. The predetermined time before the start time candidate of the drink intake is time just after a variation per a unit section before the start time of the drink intake exceeds a predetermined value (for example, 0.1 or 1).

Next, the baseline feature calculation unit 24 calculates the baseline of the period before the increasing, as a value corresponding to an average $\mu_u$ of the heart rate of the period before the increasing (Step S52). Next, the baseline feature calculation unit 24 determines the period after the decreasing (Step S53). For example, the period after the decreasing is a period from the time of the minimum heart rate to a predetermined time after the time of the minimum heart rate. The predetermined time may be two minutes or 30 minutes, for example. The predetermined time after the time of the minimum heart rate is time just before a variation per a unit section after the time of the minimum heart rate exceeds a predetermined value (for example, 0.1 or 1). Next, the baseline feature calculation unit 24 calculates the baseline of the period after the decreasing, as a value corresponding to an average $\mu_d$ of the heart rate of the period after the decreasing (Step S54).

Next, as the baseline ratio between the period before the increasing and the period after the decreasing, the baseline feature calculation unit 24 calculates a ratio $\mu_d/\mu_u$ between the baseline of the period before the increasing and the baseline of the period after the decreasing (Step S55). A value indicating a difference between before the increasing and after the decreasing may be used instead of the baseline ratio. And so, the baseline feature calculation unit 24 may calculate a difference between the baseline of the period before the increasing and the baseline of the period after the decreasing ($|\mu_u| - |\mu_d|$) as the baseline feature ix. The baseline feature calculation unit 24 calculates at least one of the baseline feature viii to the baseline feature ix.

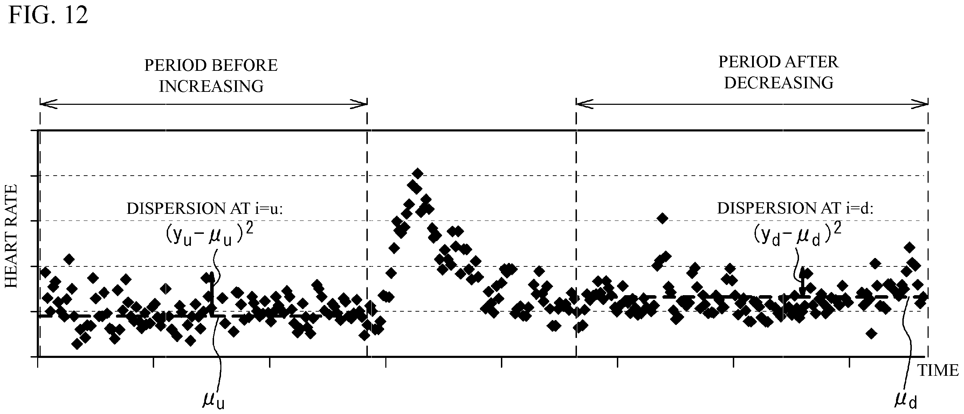

Next, the heart rate fluctuation has the tendency in which a variation (dispersion) of the heart rate after the drink intake is larger than the variation of the heart rate before the drink intake. Therefore, as illustrated in FIG. 5, the fluctuation feature calculation unit 25 calculates a fluctuation feature of the heart rate fluctuation (Step S15). In concrete, as the fluctuation feature, the fluctuation feature calculation unit 25 calculates a fluctuation of the period before the increasing (fluctuation feature xi), a fluctuation of the period after the decreasing (fluctuation feature xii) and a ratio between the fluctuation of the period before the increasing and the fluctuation of the period after the decreasing (fluctuation ratio between the period before the increasing and the period after the decreasing: fluctuation feature xiii). FIG. 11B illustrates an example of a flowchart of a calculation process of the fluctuation feature. FIG. 12 illustrates a calculation of the fluctuation feature.

As illustrated in FIG. 11B, the fluctuation feature calculation unit 25 calculates the fluctuation of the period before the increasing as the dispersion of the heart rate before the increasing (Step S61). For example, the fluctuation of the period before the increasing is a value corresponding to a dispersion $V_u$ from the average $\mu_u$ of the heart rate of the period before the increasing. The dispersion Vu may be expressed by the following formula (5).
[Formula 5]

$$V_u = \sum_{i=1}^{Q_u} (y_i - \mu_u)^2 / Q_u \quad (5)$$

Next, the fluctuation feature calculation unit 25 calculates the fluctuation of the period after the decreasing as a dispersion of the heart rate of the period after the decreasing (Step S62). For example, the fluctuation of the period after the decreasing is a value corresponding to a dispersion $V_d$ from the average $\mu_d$ of the heart rate of the period after the decreasing. The dispersion $V_d$ may be expressed by the following formula (6).
[Formula (6)]

$$V_d = \sum_{i=1}^{Q_d} (y_i - \mu_d)^2 / Q_d \quad (6)$$

Next, the fluctuation feature calculation unit 25 calculates a ratio $V_u/V_d$ between the period before the increasing and the period after the decreasing as the fluctuation ratio between the period before the increasing and the period after the decreasing (Step S63). A value indicating a difference between before the increasing and after the decreasing may be used instead of the ratio. And so, the fluctuation feature calculation unit 25 may calculate a difference ($|V_u|-|V_d|$) between the fluctuation of the period before the increasing and the fluctuation of the period after the decreasing, as the fluctuation feature xiii. The fluctuation feature calculation unit 25 calculates at least one of the fluctuation feature xi to the fluctuation feature xiii.

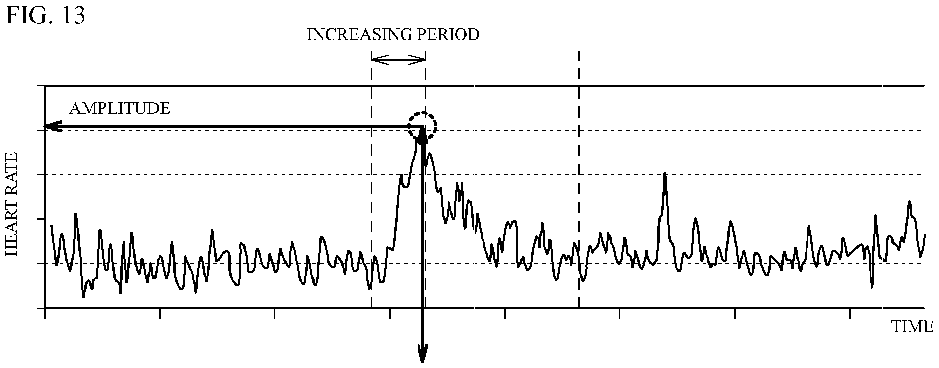

Next, in the heart rate fluctuation caused by the drink intake, an increasing width of the heart rate may be used as a feature. And so, as illustrated in FIG. 5, the amplitude feature calculation unit 26 calculates an amplitude feature (Step S16). In concrete, as illustrated in FIG. 13, as an amplitude feature xiv, the amplitude feature calculation unit 26 calculates an increasing width of the maximum heart rate of the increasing period. FIG. 11C illustrates an example of a flowchart of a calculation process of the amplitude feature xiv. As illustrated in FIG. 11C, the amplitude feature calculation unit 26 calculates a heart rate at the time of the maximum heart rate (Step S71). For example, when the heart rate = zero is a standard, a value of the maximum heart rate may be used as the amplitude feature xiv. Alternatively, when a heart rate at the start time candidate of the drink intake is a standard, a difference between the maximum heart rate and the heart rate at the start time candidate of the drink intake may be used as the amplitude feature xiv.

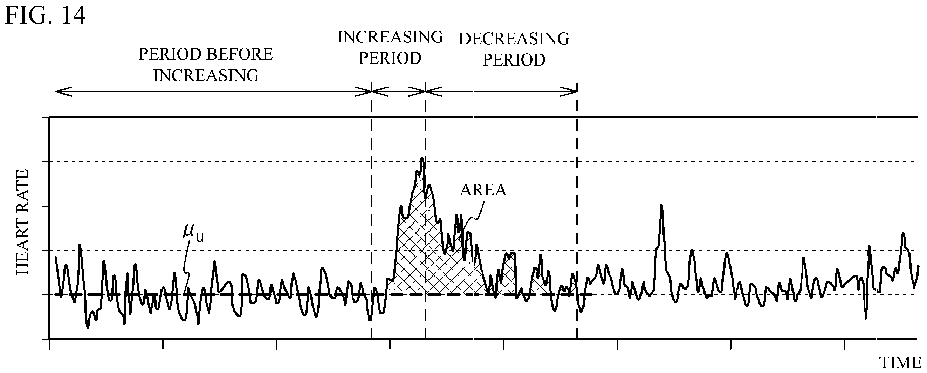

Next, in the heart rate fluctuation caused by the drink intake, an area of the heart rate (integral value) may be used as a feature. And so, as illustrated in FIG. 5, an area feature calculation unit 27 calculates an area feature (Step S17). In concrete, as illustrated in FIG. 14, as an area feature xv, a total (integral value) of the heart rate increasing width of the increasing period of the heart rate and the decreasing period of the heart or a time-averaged heart rate increasing width. The heart rate increasing width may be an increasing width from the average $\mu_u$ of the heart rate of the period before the increasing. FIG. 11D illustrates an example of a flowchart of a calculation process of the area feature xv. As illustrated in FIG. 11D, the area feature calculation unit 27 calculates the area feature xv by calculating a total value of increasing widths from the baseline of the period before the increasing from the start time candidate of the drink intake to the time of the minimum heart rate (Step S81).

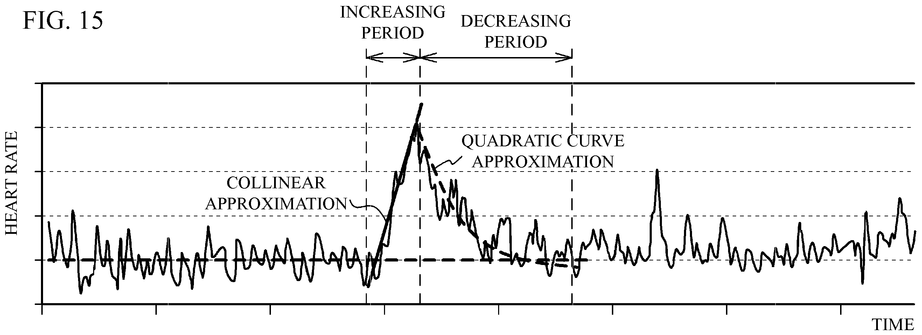

A collinear approximation is used for the calculation of the speed feature i to the speed feature iii and the difference feature iv to the difference feature vi. However, a curve approximation such as a quadratic curve may be used. However, it is preferable that the collinear approximation is used in the increasing period because the heart rate of the increasing period tends to rapidly increase. It is preferable that the curve approximation is used in the decreasing period because the heart rate of the decreasing period tends to slowly decrease. And so, as illustrated in FIG. 15, the collinear approximation may be used in the increasing period, and the curve approximation is used in the decreasing period.

The storage 40 stores a model that is made in advance. The model includes a model of the drink intake and a model of a drink non-intake. The model of the drink intake includes the speed features i to iii, the difference features iv to vi, the intersection feature vii, the baseline features viii to x, the fluctuation feature xi to xiii, the amplitude feature xiv and the area feature xv, in the heart rate fluctuation caused by the drink intake. The model of the drink non-intake includes the speed features i to iii, the difference features iv to vi, the intersection feature vii, the baseline features viii to x, the fluctuation feature xi to xiii, the amplitude feature xiv and the area feature xv, in the heart rate fluctuation during a period in which a drink is not taken. These models may be made with use of measured values of a specific object using the drink intake detection process or with use of measured values of many and unspecified users. When a plurality of measured values are used, an average of the measured values may be used. Alternatively, an analyzer may optionally make the model of the drink intake and the mode of the drink non-intake.

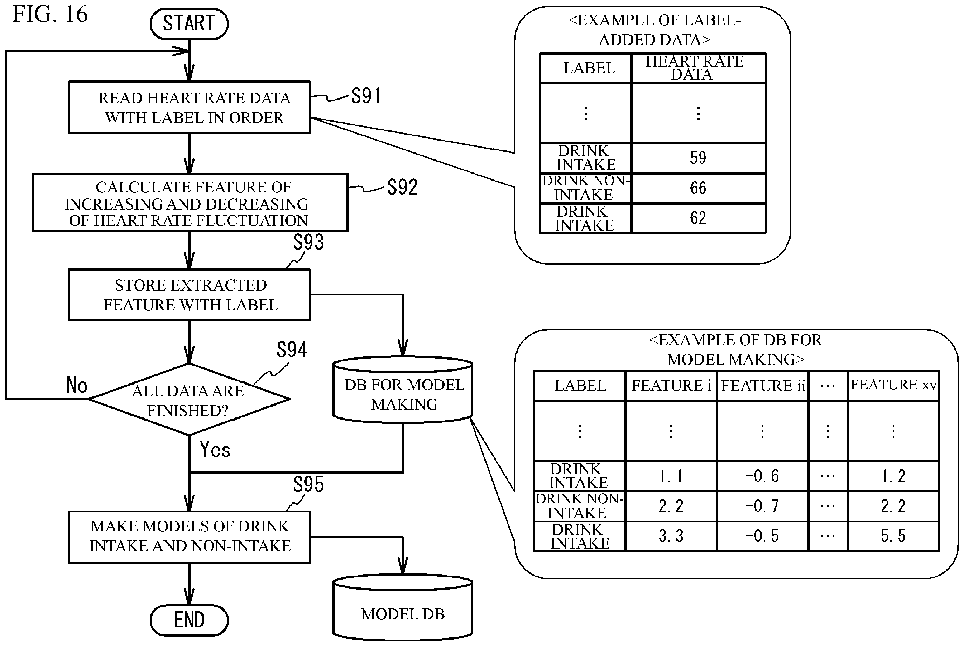

FIG. 16 illustrates an example of a flowchart for making a model stored in the storage 40. In the example, a model is made in advance with use of a measured value of a heart rate of an object using the drink intake detection process. As illustrated in FIG. 16, the heart rate acquisition unit 10 reads, in order, heart rate data to which a label is added (Step S91). For example, the label may be made by pushing a button at timing when the object takes a drink.

Next, the heartbeat fluctuation calculation unit 20 calculates a feature associated with increasing and decreasing of the heart rate fluctuation with respect to time (Step S92). The feature includes the speed features i to iii, the difference features iv to vi, the intersection feature iii, the baseline features viii to x, the fluctuation features xi to xiii, the amplitude feature xiv and the area feature xv. Next, the heartbeat fluctuation calculation unit 20 stores the calculated feature in the storage 40 as a database for model making, together with the label (Step S93). Next, the determination unit 30 determines whether the process is finished with respect to all data (Step S94). When it is determined as "No" in Step S94, Step S91 is executed again. When it is determined as "Yes" in Step S94, the determination unit 30 makes a model of the drink intake and a model of the drink non-intake, with use of the database for model making (Step S95). These models are stored in the storage 40 as a model used for the drink intake detection process.

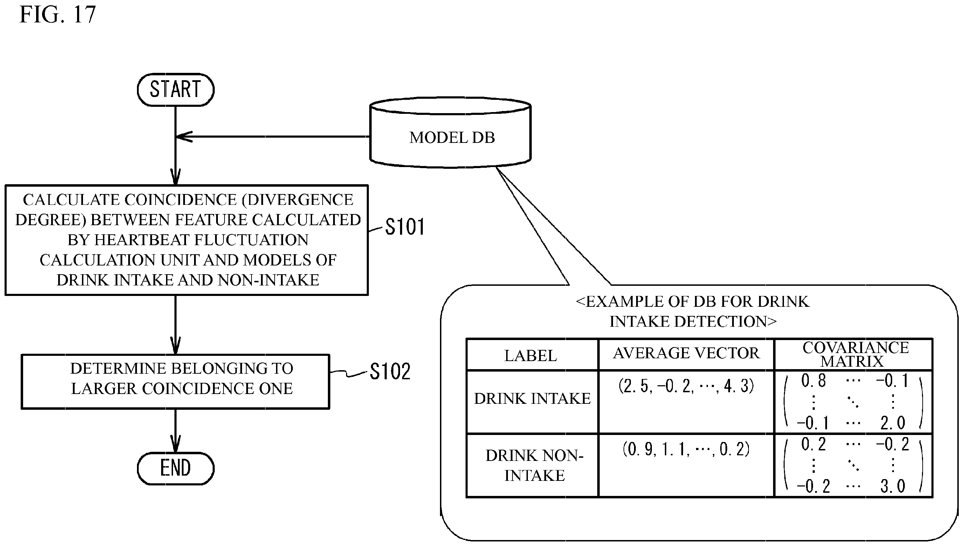

Next, as illustrated in FIG. 3, the determination unit 30 determines whether the fluctuation of the heart rate during the drink intake detection process is caused by a drink intake, with use of the feature calculated by the heartbeat fluctuation calculation unit 20 (Step S3). FIG. 17 illustrates an example of a flowchart of a determination process in this case. As illustrated in FIG. 17, the determination unit 30 calculates a coincidence degree (divergence degree) between the feature calculated from the heart rate fluctuation measured during the drink intake detection process and the model of the drink intake stored in the storage 40 and between the feature and the model of the drink non-intake stored in the storage 40 (Step S101). The coincidence degree indicates a similarity degree between the feature calculated from the heart rate fluctuation measured during the drink intake detection process and the model of the drink intake or a similarity degree between the feature and the model of the drink non-intake. Therefore, the coincidence means a similarity degree or an analogous degree. In the following, a Mahalanobis distance is described as a coincidence calculation. However, any algorism such as a support vector machine, a boosting or a neural network may be applied to the coincidence calculation.

For example, the determination unit 30 uses the Mahalanobis distance for calculating the coincidence degree (divergence degree). The determination unit 30 uses an average vector and a covariance matrix as parameters when calculating the Mahalanobis distance. The average vector and the covariance matrix of the drink intake are $\mu_d$ and $\Sigma_d$. The average vector and the covariance matrix of the drink non-intake are $\mu_n$ and $\Sigma_n$. Data to be determined is $x=(x_1, x_2, ...)^T$. $x_1$, $x_2$, ... indicate the feature i, the feature ii, $\cdots$. The Mahalanobis distance may be expressed by the following formula (7) and the following formula (8). When the covariance matrix is a diagonal matrix, a normalized Euclid distance (that is normalized so that dispersions of features are equal to each other) may be used. The covariance matrix is a unit matrix, an Euclid distance may be used.

[Formula 7]

$$D_d = \sqrt{(x - \mu_d)^T \sum_d{}^{-1} (x - \mu_d)} \quad (7)$$

[Formula 8]

$$D_n = \sqrt{(x - \mu_n)^T \sum_n{}^{-1} (x - \mu_n)} \quad (8)$$

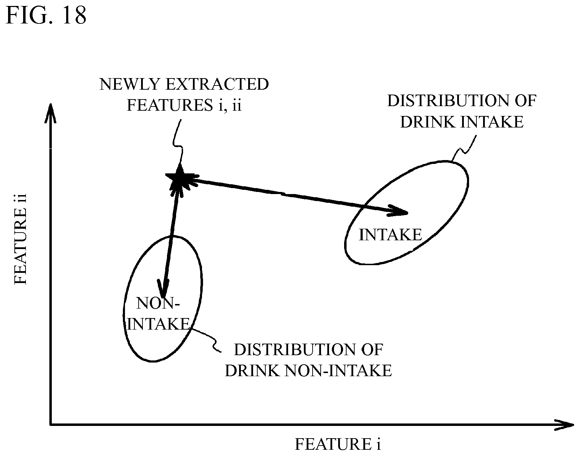

Next, the determination unit 30 determines that the heart rate fluctuation measured during the drink intake detection process belongs to one of which the distance is smaller (Step S102). Therefore, when $D_d$ is larger than $D_n$ as illustrated in FIG. 18, the determination unit 30 determines that the heart rate fluctuation measured during the drink intake detection process is the heart rate fluctuation of the drink non-intake. In FIG. 18, a distance using only the features i and ii is illustrated for simplification. Information associated with a viscosity of a liquid may be estimated with use of $D_d$ and $D_n$, and the estimated information may be added to the determination result of the determination unit 30. For example, when $D_d$ is smaller than $D_n$ and a ratio of $D_d$ and $D_n$ is equal to a predetermined value (for example, 2) or less, the determination unit 30 determines that the heart rate fluctuation measured during the drink intake detection process is the heart rate fluctuation of the intake of a drink having a low viscosity. Alternatively, a distance from not the model of the drink non-intake but a plurality of models of the drink intake may be calculated, a component of the drink may be estimated, and the calculated distance and the estimated component may be added to the determination result of the determination unit 30. For example, a distance from a model of pure water intake, a model of intake of a drink including salt, and a model of intake of a drink including sugar may be used, an including amount of the salt and the sugar may be estimated, and it may be determined that the heart rate fluctuation is that of the intake of the drink including 10 mg of salt and 10 mg of sugar.

In accordance with the embodiment, it is determined whether a heart rate fluctuation of an object with respect to time is caused by a drink intake, on a basis of a coincidence degree between a feature extracted from the heart rate fluctuation of the object and a feature of a drink intake stored in a database. It is therefore possible to detect a drink intake with high accuracy. It is possible to detect a drink intake by only acquiring heart rate fluctuation. The heart rate fluctuation is measured. Therefore, it is not necessary for the object to perform a specific action before and after a drink intake. When a wearable device put on an arm for measuring a heart rate is used, it is possible to easily detect a drink intake with low load.

When a coincidence degree with the feature of the drink intake stored in the database is higher than a coincidence degree with the feature of the drink non-intake stored in the database, it is determined that the heart rate fluctuation is caused by a drink intake. Therefore, the detection accuracy of the drink intake is improved. It may not be necessarily calculated the coincidence degree with the feature of the drink non-intake. For example, when the coincidence degree with the feature of the drink intake stored in the database is equal to or more than a threshold, it may be determined that the heart rate fluctuation is caused by a drink intake. For example, in Step S102 of FIG. 17, the coincidence degree is compared with a threshold. At least one of the above-mentioned features is used. When the features i to iii, the difference features iv to vi and the intersection feature vii are used, the detection accuracy of a drink intake becomes specifically high.

It is possible to advice life style habit with the determination result of the determination unit 30, by adding an action such as exercise and sleep. For example, when no drink is taken for a predetermined period after getting up, it is displayed "please take a drink after getting up". Alternatively, when no drink is taken at a predetermined time, it is displayed "You may forget taking a medicine".

The determination result may include at least one of the number of drink intake, the start time of the drink intake, the end time of the drink intake, the viscosity of the liquid, the component of the drink, and the amount of taken drink. The number of drink intake corresponds to the number of the determination as drink intake for a day. The start time of the drink intake, for example, corresponds to the start time candidate of the drink intake. The end tie of the drink intake, for example, corresponds to the time of the maximum heart rate. The amount of taken drink, for example, corresponds to a value obtained by dividing the period of the drink intake (difference between the end time of the drink intake and the start time of the drink intake) and a period for performing a single swallowing of the drink. The period for performing a single swallowing is stored in the database in advance, and may be made with respect to age groups, sex groups, or individuals.

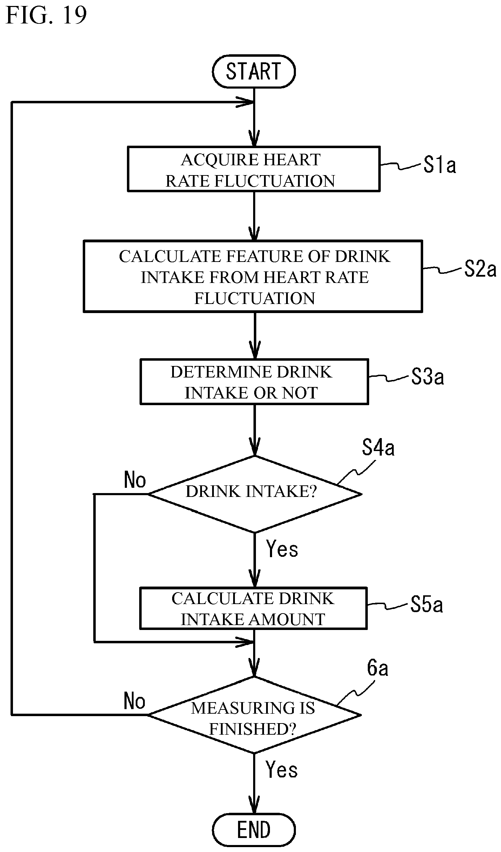

FIG. 19 illustrates an example of a flowchart of a calculation of the amount of taken drink. The heart rate acquisition unit 10 acquires the heart rate fluctuation by acquiring the heartbeat from the heartbeat measuring device 105 (Step S1a). Next, the heartbeat fluctuation calculation unit 20 calculates a feature of the drink intake in the heart rate fluctuation acquired by the heart rate acquisition unit 10 (Step S2a). Next, the determination unit 30 determines whether the heart rate fluctuation during the drink intake detection process is caused by the drink intake, with use of the feature calculated by the heartbeat fluctuation calculation unit 20 (Step S3a). Next, when it is determined as the drink intake (Yes in Step S4a), the determination unit 30 calculated the amount of taken drink (Step S5a). After Step S5a or it is not determined as the drink intake in Step S3a (No in Step S4a), the determination unit 30 determines whether the measuring is finished (Step S6a). When it is determined as "No" in Step S6a, Step S1a is executed again. When it is determined as "Yes" in Step S6a, the flowchart is terminated.

(Second Embodiment)

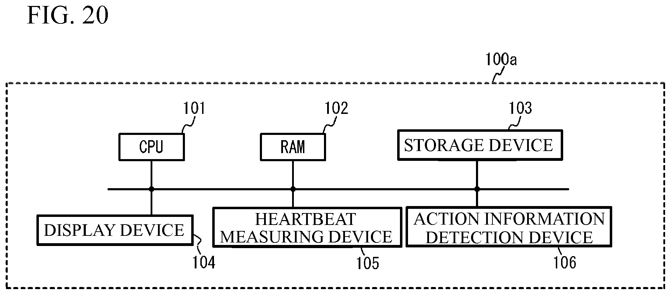

A second embodiment is an example in which action information of an object has an influence on the drink intake detection process. FIG. 20 illustrates a block diagram of a hardware structure of an information processing device 100a in accordance with the second embodiment. As illustrated in FIG. 20, the information processing device 100a further has an action information detection device 106. The action information detection device 106 is a device for detecting action information of the object.

The action information detection device 106 detects an action having a low correlation with the drink intake. For example, the action information detection device 106 detects an action in which no drink is physically taken. In concrete, the action information detection device 106 detects such as sleep, phonation, chewing, or sigh. The action information detection device 106 detects an action in which the drink is not taken with high possibility. In concrete, the action information detection device 106 detects a general habit. For example, the action information detection device 106 detects an action in which the object is grounded and moves his legs, such as walking, running or stairs stepping. The action information detection device 106 detects an action in which the object moves his hand, such as dishwashing or cleaning. The action information detection device 106 detects an action for riding on a specific vehicle such as a motorcycle. The action information detection device 106 detects an action of excretion. The action information detection device 106 detects an action of bathing. The action information detection device 106 detects a habit of an individual. For example, the action information detection device 106 detects such as talking with a specific person, meeting, a case where the object doesn't desire to urinate (action of lining up).

Alternatively, the action information detection device 106 detects an action having a high correlation with the drink intake. For example, the action information device 106 detects an action unique to the drink intake (motion of a body part such as an arm or breast of which an inclination changes according the drink intake). In concrete, the action information detection device 106 detects such as preliminary motion of putting liquid in a container, a rotation of hand of opening a cap of a PET bottle, a motion of front and behind or up and down for moving a container to a mouth, a stationary condition for a period of holding a container in his mouth and swallowing, a motion of behind and down of swallowing liquid in a container, or a motion front and behind or up and down of putting a container on a desk or the like. The action information detection device 106 detects an action in which the object takes a drink with high possibility. For example, the action information detection device 106 detects a general habit. For example, the action information detection device 106 detects such as an action of taking a drink after getting up and before sleeping, an action of taking a drink before or after bathing, or an action of taking a drink before or after exercise. The action information detection device 106 detects a habit of an individual. For example, the action information detection device 106 detects an action of taking water at a specific time (for example, 15:00), or an action of taking water in a specific place (for example, kitchen). The action information detection device 106 may determine the action with use of a motion data or determine time and place with use of data associated with time or place, with use of a conventional technology or with use of a label that the object makes by pushing a button during each motion.

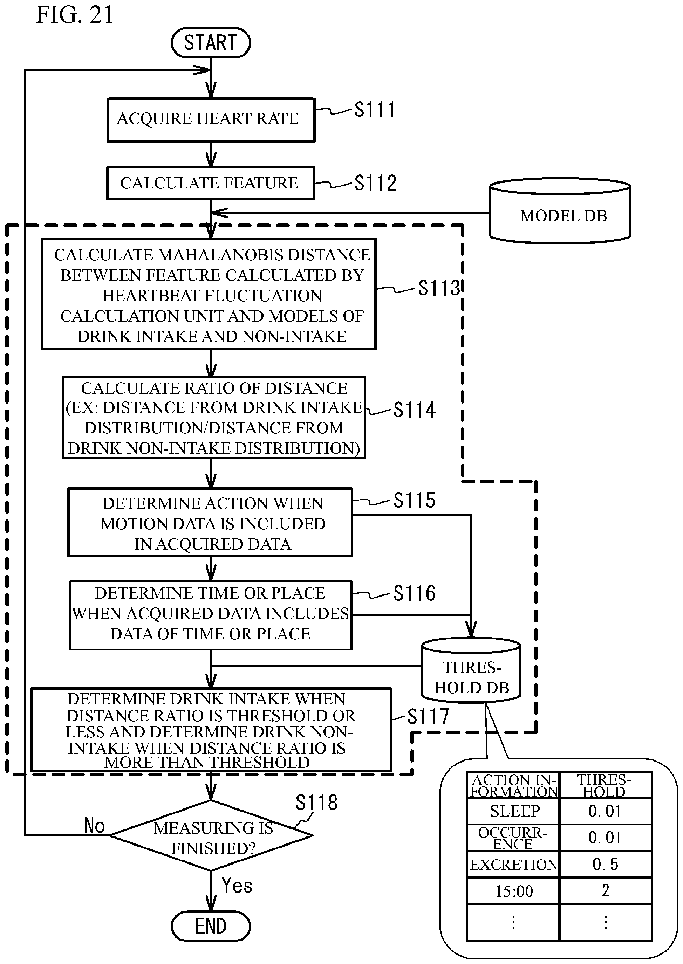

FIG. 21 illustrates an example of a flowchart of the drink intake detection process of the embodiment. As illustrated in FIG. 21, the heart rate acquisition unit 10 acquires a heartbeat from the heartbeat measuring device 105 and acquires the heart rate fluctuation with respect to time (Step Sill). The heart rate acquisition unit 10 acquires action information of the object together with the heart rate fluctuation with respect to time when the action information detection device 106 detects an action of the object. Next, the heartbeat fluctuation calculation unit 20 calculates a feature of the drink intake in the heart rate fluctuation acquired by the heart rate acquisition unit 10 (Step S112).

Next, the determination unit 30 calculates a Mahalanobis distance between the feature calculated by the heartbeat fluctuation calculation unit 20 and the model of the drink intake and between the feature and the model of the drink non-intake (Step S113). Next, the determination unit 30 calculates a ratio of the distances (Step S114). The ratio may be a distance from the model of the drink intake / a distance from the model of the drink non-intake.

Next, when the heart rate fluctuation acquired by the heart rate acquisition unit 10 includes a motion data of the object, the determination unit 30 determines an action associated with the motion data (Step S115). Next, when the heart rate fluctuation acquired by the heart rate acquisition unit 10 includes a data associated with time or a place of the object, the determination unit 30 determines the time and the place (Step S116).

Next, the determination unit 30 refers to thresholds stored in the storage 40 in advance, and acquires a threshold associated with an action detected by the action information detection device 106. When the distance ratio calculated in Step S114 is equal to or less than a threshold, the determination unit 30 determines that a drink is not taken (Step S117). Next, the determination unit 30 determines whether the measuring is finished (Step S118). When it is determined as "No" in Step S118, Step Sill is executed again. When it is determined as "Yes" in Step S118, the flowchart is terminated.

In accordance with the embodiment, the detection accuracy of the drink intake is improved because the action information of the object has an influence on the determination whether the heart rate fluctuation of the object is caused by the drink intake or not. For example, when the threshold of the distance ratio calculated in Step S114 in a case where the possibility of the drink intake is low, it is possible to suppress a false detection of the drink intake. When the threshold of the distance ration calculated in Step S114 in a case where the possibility of the drink intake is high, it is possible to suppress a false detection of the drink intake.

(Third Embodiment)

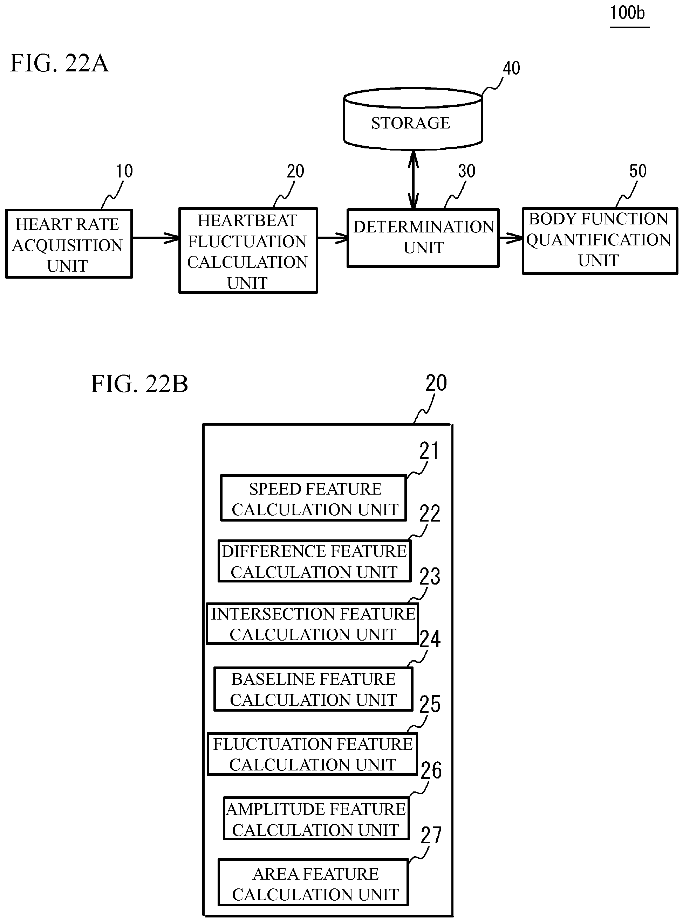

FIG. 22A illustrates a functional block diagram of an information processing device 100b in accordance with a third embodiment. The function is implemented by execution of the information processing program. As compared with the first embodiment, a body function quantification unit 50 is further implemented. FIG. 22B illustrates a functional block diagram of each function of the heartbeat fluctuation calculation unit 20. Each function of the heartbeat fluctuation calculation unit 20 is the same as the first embodiment. The body function quantification unit 50 quantifies the body function by comparing the feature obtained from the heart rate fluctuation acquired in the drink intake detection process with the model of the drink intake. For example, the body function quantification unit 50 determines that the object is thirst twice as usual when an acquired increasing speed of the heart rate is twice as the increasing speed of the model. The body function quantification unit 50 determines that function of the body of the object becomes weak when an acquired decreasing speed of the heart rate is half of the decreasing speed of the model.

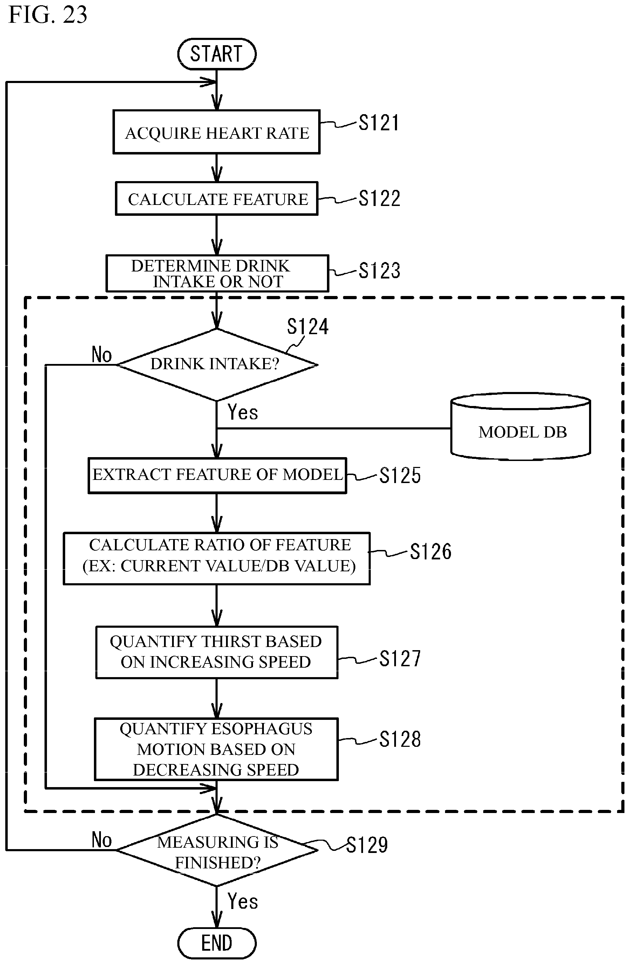

FIG. 23 illustrates an example of a flowchart of a drink intake detection process in accordance with the embodiment. As illustrated in FIG. 23, the heart rate acquisition unit 10 acquires the heart rate fluctuation by acquiring a heartbeat from the heartbeat measuring device 105 (Step S121). Next, the heartbeat fluctuation calculation unit 20 calculates a feature of the drink intake in the heart rate fluctuation acquired by the heart rate acquisition unit 10 (Step S122). Next, the determination unit 30 determines the drink intake or the drink non-intake, with use of the feature calculated in Step S122 (Step S123).

Next, the body function quantification unit 50 determines whether it is determined as "drink intake" in Step S123 (Step S124). When it is determined as "Yes" in Step S124, the body function quantification unit 50 refers to the storage 40 and acquires each feature of the model of the drink intake (Step S125). When the model of the drink intake includes a plurality of features, the body function quantification unit 50 may an average of the features.

Next, the body function quantification unit 50 calculates a ratio of each feature calculated in Step S122 and each feature acquired in Step S125 (Step S126). Next, the body function quantification unit 50 quantifies the thirsty, in accordance with the increasing speed of the heart rate (Step S127). Next, the body function quantification unit 50 quantifies the motion of the body, in accordance with the decreasing speed of the heart rate (Step S128). The body function quantification unit 50 determines whether the measuring is finished (Step S129). When it is determined as "Yes" in Step S129, the flowchart is terminated. When it is determined as "No" in Step S129, Step S121 is executed again. When it is determined as "No" in Step S124, Step S129 is executed again.

In accordance with the embodiment, it is possible to quantify the body function of the object by using a relationship between the feature of he heart rate fluctuation of the object and the feature stored in the database.

(Another feature)

In the above-mentioned embodiments, the speed feature, the difference feature and so on of the heart rate fluctuation are used. However, the feature is not limited. For example, a wave pattern of the heart rate fluctuation may be used as the feature. In concrete, when a coincidence degree (divergence degree) between a wave pattern of the heart rate of the object and a wave pattern of the drink intake that is stored in the storage 40 in advance is high, it may be determined that the case is the drink intake.

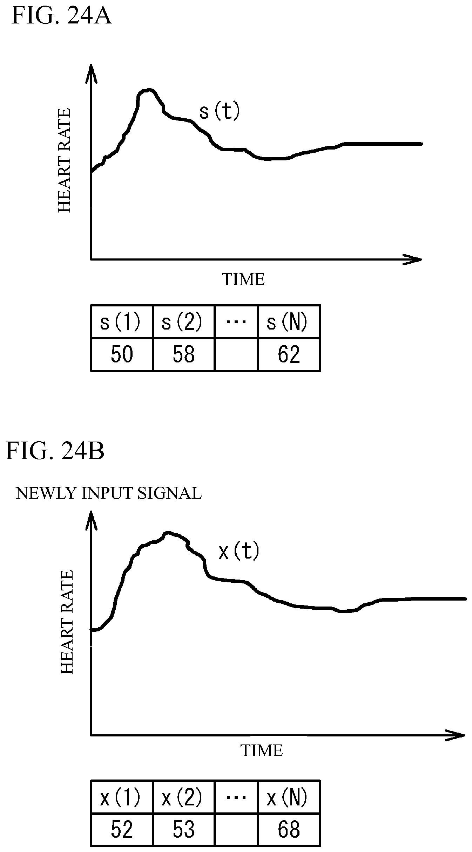

A heart rate fluctuation signal with respect to time stored in the storage 40 is s(t). A heart rate fluctuation signal acquired in the drink intake detection process is x(t). FIG. 24A illustrates s(t). FIG. 24B illustrates x(t). In this case, a coincidence between the patterns may be expressed by the following formula (9). In the following formula (9), a difference is calculated. And, the smaller the value is, the high the coincidence is. N is a total number of the signals. The number of the signals are the number of signals within seconds from the start time of the period before increasing to the end time of the period after the decreasing. When the coincidence is equal to or more than a threshold set in advance, it may be determined that the case is the drink intake. When the number of the signals is different from the total number N of the signals, the signals may be interpolated or thinned out.

[Formula 9]

$$\|s - x\| = \sqrt{\sum_{j=1}^{N} \left(s(t_j) - x(t_j)\right)^2} \quad (9)$$

(Another device configuration)

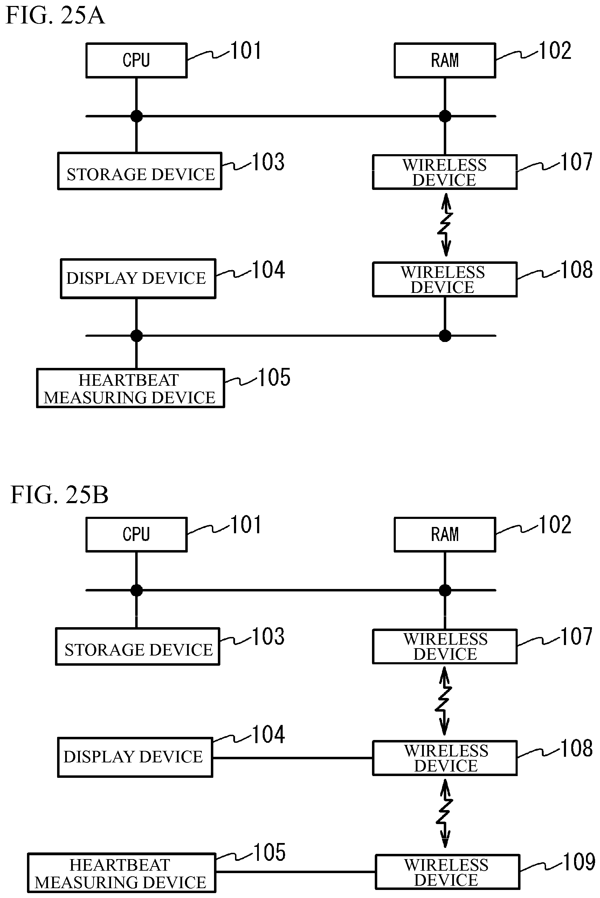

FIG. 25A and FIG. 25B illustrate another device configuration of the information processing device 100. As illustrated in FIG. 25A, the information processing device 100 may have a structure in which a server wirelessly sends data to a wearable device and wirelessly receives data from the wearable device. The server has the CPU 101, the RAM 102, the storage device 103 and a wireless device 107. The wearable device has the display device 104, the heartbeat measuring device 105, and a wireless device 108. Alternatively, as illustrated in FIG. 25B, the information processing device may have a structure in which data is wirelessly sent or received among a server, a terminal and a wearable device. In this case, the server has the CPU 101, the RAM 102, the display device 103 and the wireless device 107. The terminal has the display device 104 and the wireless device 108. The wearable device has the heartbeat measuring device 105 and a wireless device 109. When the action information detection device 106 is provided, the wearable device may have the action information detection device 106.

The heart rate acquisition unit 10 acts as an example of a heart rate acquisition unit configured to acquire a heart rate of an object. The heartbeat fluctuation calculation unit 20 acts as an example of a feature extraction unit configured to extract a feature from heart rate fluctuation with respect to time that is acquired by the heart rate acquisition unit. The storage 40 acts as an example of a storage configured to, in advance, store information associated with heart rate fluctuation of a drink intake. The determination unit 30 acts as an example of a determination unit configured to determine whether the heart rate fluctuation acquired by the heart rate acquisition unit is caused by a drink intake, on a basis of the feature extracted by the feature extraction unit and the information stored in the storage. The action information detection device 106 acts as an example of an action information measuring device configured to measure action information of the object. The body function quantification unit 50 acts as an example of a quantification unit configured to quantify a body function of the object on a basis of a relationship between the feature extracted by the feature extraction unit and the information stored in the storage when the determination unit determines that the heart rate fluctuation acquired by the heart rate acquisition unit is caused by the drink intake.

Embodiments of the present invention have been described in detail. However, the present invention is not limited to the specific embodiments but may be varied or changed within the scope of the claimed invention.

**Claims**

1. An information processing device (100) comprising:

    a heart rate acquisition unit (10) configured to acquire a heart rate of an object;
    a feature extraction unit (20) configured to extract a feature from heart rate fluctuation with respect to time that is acquired by the heart rate acquisition unit (10), the feature including an average and a variation before an increasing of the heart rate is detected and an average and a variation after the heart rate returns to a predetermined value after the increasing;
    a storage (40) configured to, in advance, store information associated with heart rate fluctuation of a drink intake; and
    a determination unit (30) configured to determine whether the heart rate fluctuation acquired by the heart rate acquisition unit (10) is caused by a drink intake, on a basis of the feature extracted by the feature extraction unit and the information stored in the storage.

2. The information processing device (100) as claimed in claim 1, **characterized in that**
    the determination unit (30) is configured to determine that the heart rate fluctuation acquired by the heart rate acquisition unit (10) is caused by the drink intake, when a coincidence between the feature extracted by the feature extraction unit and a feature extracted from the information stored in the storage is equal to a threshold or more.

3. The information processing device (100) as claimed in claim 1, **characterized in that**:

    the storage (40) is configured to, in advance, store information associated with heart rate fluctuation with respect to time of a drink non-intake; and
    the determination unit (30) is configured to determine that the heart rate fluctuation acquired by the heart rate acquisition unit (10) is caused by the drink intake, when a coincidence between the feature extracted by the feature extraction unit and a feature extracted from the information stored in the storage is larger than a coincidence between the feature extracted by the feature extraction unit and a heart rate feature of the drink non-intake extracted from the information stored in the storage.

4. The information processing device (100) as claimed in any of claims 1 to 3, **characterized in that**
    the coincidence is calculated on a basis of one of an Euclid distance, a normalized Euclid distance and a Mahalanobis distance.

5. The information processing device (100) as claimed in any of claims 1 to 4, **characterized by** further comprising:

an action information measuring device (106) configured to measure action information of the object, wherein the determination unit (30) is configured to use the action information measured by the action information measuring device when determining whether the heart rate fluctuation acquired by the heart rate acquisition unit (10) is caused by the drink intake.

6. The information processing device (100) as claimed in any of claims 1 to 5, **characterized by** further comprising: a quantification unit (50) configured to quantify a body function of the object on a basis of a relationship between the feature extracted by the feature extraction unit (20) and the information stored in the storage (40) when the determination unit (30) determines that the heart rate fluctuation acquired by the heart rate acquisition unit (10) is caused by the drink intake.

7. The information processing device (100) as claimed in any of claims 1 to 6, **characterized by** further comprising: a drink intake amount estimation unit (30) configured to estimate a drink intake amount with use of the feature extracted by the feature extraction unit and a period for one swallowing of a drink that is stored in the storage in advance, when the determination unit determines that the heart rate fluctuation acquired by the heart rate acquisition unit (10) is caused by the drink intake.

8. The information processing device (100) as claimed in any of claims 1 to 6, **characterized in that** the feature includes at least one of an increasing speed of heart rates and a decreasing speed of heart rates.

9. The information processing device (100) as claimed in any of claims 1 to 6, **characterized in that** the feature includes an error between heart rate fluctuation with respect to time and an approximated line of the heart rate fluctuation.

10. The information processing device (100) as claimed in any of claims 1 to 6, **characterized in that** the feature includes an intersection angle between an approximated line of heart rate fluctuation before a predetermined time and another approximated line of the heart rate fluctuation after a time of a maximum heart rate.

11. The information processing device (100) as claimed in claim 10, **characterized in that** the predetermined time is the time of the maximum heart rate.

12. The information processing device (100) as claimed in any of claims 1 to 6, **characterized in that** the feature includes a value based on a peak value of the heart rate.

13. The information processing device (100) as claimed in any of claims 1 to 6, wherein the feature includes an integral value from an increasing of the heart rate to returning to a predetermined value after the increasing.

14. The information processing device (100) as claimed any of claims 1 to 6, **characterized in that** the feature includes a wave pattern of the heart rate fluctuation.

15. The information processing device (100) as claimed in any of claims 1 to 6, **characterized in that** the feature includes one or more of an increasing speed of the heart rate, a decreasing speed of the heart rate, an error between heart rate fluctuation and an approximated line of the heart rate fluctuation, an intersection angle between an approximated line of the heart rate fluctuation before a time of the maximum heart rate and another approximated line of the heart rate fluctuation after the time of the maximum heart rate, a value based on a peak value of the heart rate, an integral value from the increasing of the heart rate to returning to a predetermined value after the increasing, and a wave pattern of the heart rate fluctuation.

16. The information processing device (100) as claimed in any of claims 1 to 6, **characterized in that** the feature includes an increasing speed of the heart rate, a decreasing speed of the heart rate, an error between the heart rate fluctuation and an approximated line of the heart rate fluctuation, and an intersection angle between an approximated line of the heart rate fluctuation before a time of a maximum heart rate and another approximated line of the heart rate fluctuation after the time of the maximum heart rate.

17. An information processing method executed by a computer, the information processing method comprising:

acquiring a heart rate of an object;
extracting a feature from heart rate fluctuation with respect to time that is acquired in the acquiring, the feature including an average and a variation before an increasing of the heart rate is detected and an average and a variation after the heart rate returns to a predetermined value after the increasing; and

determining whether the heart rate fluctuation acquired in the acquiring is caused by a drink intake, on a basis of the feature extracted in the extracting and information stored, in advance, in a storage storing the information associated with heart rate fluctuation of a drink intake.

18. An information processing program which causes the computer to carry out the information processing method according to claim 17.

**Patentansprüche**

1. Informationsverarbeitungsvorrichtung (100), umfassend:

eine Herzfrequenzerfassungseinheit (10), die konfiguriert ist, eine Herzfrequenz eines Objekts zu erfassen;
eine Merkmalsextraktionseinheit (20), die konfiguriert ist, ein Merkmal in Bezug auf die Zeit aus einer Herzfrequenzfluktuation zu extrahieren, die von der Herzfrequenzerfassungseinheit (10) erfasst wird, wobei das Merkmal einen Mittelwert und eine Variation, bevor eine Zunahme der Herzfrequenz detektiert wird, und einen Mittelwert und eine Variation, nachdem die Herzfrequenz nach der Zunahme auf einen vorbestimmten Wert zurückgekehrt ist, aufweist;
einen Speicher (40), der konfiguriert ist, vorab Informationen zu speichern, die der Herzfrequenzfluktuation einer Getränkeeinnahme zugeordnet sind; und
eine Bestimmungseinheit (30), die konfiguriert ist, zu bestimmen, ob die von der Herzfrequenzerfassungseinheit (10) erfasste Herzfrequenzfluktuation durch eine Getränkeeinnahme verursacht wird, basierend auf dem von der Merkmalsextraktionseinheit extrahierten Merkmal und den in dem Speicher gespeicherten Informationen.

2. Informationsverarbeitungsvorrichtung (100) nach Anspruch 1, **dadurch gekennzeichnet, dass** die Bestimmungseinheit (30) konfiguriert ist, zu bestimmen, dass die von der Herzfrequenzerfassungseinheit (10) erfasste Herzfrequenzfluktuation durch die Getränkeeinnahme verursacht wird, wenn eine Koinzidenz zwischen dem durch die Merkmalsextraktionseinheit extrahierten Merkmal und einem aus den in dem Speicher gespeicherten Informationen extrahierten Merkmal gleich einem Schwellenwert oder höher ist.

3. Informationsverarbeitungsvorrichtung (100) nach Anspruch 1, **dadurch gekennzeichnet, dass**:

der Speicher (40) konfiguriert ist, vorab Informationen zu speichern, die einer Herzfrequenzfluktuation in Bezug auf die Zeit einer Getränke-Nichteinnahme zugeordnet sind; und
die Bestimmungseinheit (30) konfiguriert ist, zu bestimmen, dass die von der Herzfrequenzerfassungseinheit (10) erfasste Herzfrequenzfluktuation durch die Getränkeeinnahme verursacht wird, wenn eine Koinzidenz zwischen dem von der Merkmalsextraktionseinheit extrahierten Merkmal und einem aus den in dem Speicher gespeicherten Informationen extrahierten Merkmal größer als eine Koinzidenz zwischen dem von der Merkmalsextraktionseinheit extrahierten Merkmal und einem aus den in dem Speicher gespeicherten Informationen extrahierten Herzfrequenzmerkmal der Getränke-Nichteinnahme ist.

4. Informationsverarbeitungsvorrichtung (100) nach einem der Ansprüche 1 bis 3, **dadurch gekennzeichnet, dass** die Koinzidenz basierend auf einem euklidischen Abstand, einem normalisierten euklidischen Abstand, oder einem Mahalanobis-Abstand berechnet wird.

5. Informationsverarbeitungsvorrichtung (100) nach einem der Ansprüche 1 bis 4, **dadurch gekennzeichnet, dass** sie ferner umfasst:

eine Aktionsinformations-Messvorrichtung (106), die konfiguriert ist, Aktionsinformationen des Objekts zu messen,
wobei die Bestimmungseinheit (30) konfiguriert ist, von der Aktionsinformations-Messvorrichtung gemessene Aktionsinformationen zu verwenden, wenn bestimmt wird, ob die von der Herzfrequenzerfassungseinheit (10) erfasste Herzfrequenzfluktuation durch die Getränkeeinnahme verursacht wird.

6. Informationsverarbeitungsvorrichtung (100) nach einem der Ansprüche 1 bis 5, **dadurch gekennzeichnet, dass** sie ferner umfasst:

eine Quantifizierungseinheit (50), die konfiguriert ist, eine Körperfunktion des Objekts basierend auf einer Beziehung zwischen dem durch die Merkmalsextraktionseinheit (20) extrahierten Merkmal und den in dem Speicher (40) gespeicherten Information zu quantifizieren, wenn die Bestimmungseinheit (30) bestimmt, dass die von der Herzfrequenzerfassungseinheit (10) erfasste Herzfrequenzfluktuation durch die Getränkeeinnahme verursacht wird.

7. Informationsverarbeitungsvorrichtung (100) nach einem der Ansprüche 1 bis 6, **dadurch gekennzeichnet, dass** sie ferner umfasst:
eine Getränke-Einnahmemengen-Schätzeinheit (30), die konfiguriert ist, eine Getränkeeinnahmemenge unter Verwendung des von der Merkmalsextraktionseinheit extrahierten Merkmals und einer Zeitdauer für ein Schlucken eines Getränks, die vorab in dem Speicher gespeichert ist, zu schätzen, wenn die Bestimmungseinheit bestimmt, dass die von der Herzfrequenzerfassungseinheit (10) erfasste Herzfrequenzfluktuation durch die Getränkeeinnahme verursacht wird.

8. Informationsverarbeitungsvorrichtung (100) nach einem der Ansprüche 1 bis 6, **dadurch gekennzeichnet, dass** das Merkmal eine zunehmende Geschwindigkeit von Herzfrequenzen und/oder eine abnehmende Geschwindigkeit von Herzfrequenzen aufweist.

9. Informationsverarbeitungsvorrichtung (100) nach einem der Ansprüche 1 bis 6, **dadurch gekennzeichnet, dass** das Merkmal einen Fehler zwischen einer Herzfrequenzfluktuation in Bezug auf die Zeit und einer angenäherten Linie der Herzfrequenzfluktuation aufweist.

10. Informationsverarbeitungsvorrichtung (100) nach einem der Ansprüche 1 bis 6, **dadurch gekennzeichnet, dass** das Merkmal einen Schnittwinkel zwischen einer angenäherten Linie der Herzfrequenzfluktuation vor einer vorbestimmten Zeit und einer anderen angenäherten Linie der Herzfrequenzfluktuation nach einer Zeit einer maximalen Herzfrequenz aufweist.

11. Informationsverarbeitungsvorrichtung (100) nach Anspruch 10, **dadurch gekennzeichnet, dass** die vorbestimmte Zeit die Zeit der maximalen Herzfrequenz ist.

12. Informationsverarbeitungsvorrichtung (100) nach einem der Ansprüche 1 bis 6, **dadurch gekennzeichnet, dass** das Merkmal einen Wert aufweist, der auf einem Spitzenwert der Herzfrequenz basiert.

13. Informationsverarbeitungsvorrichtung (100) nach einem der Ansprüche 1 bis 6, wobei das Merkmal einen Integralwert von einer Zunahme der Herzfrequenz bis zu einem Zurückkehren auf einen vorbestimmten Wert nach der Zunahme aufweist.

14. Informationsverarbeitungsvorrichtung (100) nach einem der Ansprüche 1 bis 6, **dadurch gekennzeichnet, dass** das Merkmal ein Wellenmuster der Herzfrequenzfluktuation aufweist.

15. Informationsverarbeitungsvorrichtung (100) nach einem der Ansprüche 1 bis 6, **dadurch gekennzeichnet, dass** das Merkmal eines oder mehreres von einer zunehmenden Geschwindigkeit der Herzfrequenz, einer abnehmenden Geschwindigkeit der Herzfrequenz, einem Fehler zwischen der Herzfrequenzfluktuation und eine angenäherte Linie der Herzfrequenzfluktuation, einem Schnittwinkel zwischen einer angenäherten Linie der Herzfrequenzfluktuation vor einem Zeitpunkt der maximalen Herzfrequenz und einer weiteren angenäherten Linie der Herzfrequenzfluktuation nach dem Zeitpunkt der maximalen Herzfrequenz, einem Wert, der auf einem Spitzenwert der Herzfrequenz basiert, einem Integralwert von der Zunahme der Herzfrequenz bis zu einem Zurückkehren auf einen vorbestimmten Wert nach der Zunahme, und einem Wellenmuster der Herzfrequenzfluktuation aufweist.

16. Informationsverarbeitungsvorrichtung (100) nach einem der Ansprüche 1 bis 6, **dadurch gekennzeichnet, dass** das Merkmal eine zunehmende Geschwindigkeit der Herzfrequenz, eine abnehmende Geschwindigkeit der Herzfrequenz, einen Fehler zwischen der Herzfrequenzfluktuation und einer angenäherte Linie der Herzfrequenzfluktuation, und einen Schnittwinkel zwischen einer angenäherten Linie der Herzfrequenzfluktuation vor einem Zeitpunkt einer maximalen Herzfrequenz und einer weiteren angenäherten Linie der Herzfrequenzfluktuation nach dem Zeitpunkt der maximalen Herzfrequenz aufweist.

17. Informationsverarbeitungsverfahren, das von einem Computer ausgeführt wird, wobei das Informationsverarbeitungsverfahren umfasst:

Erfassen einer Herzfrequenz eines Objekts;

Extrahieren eines Merkmals aus einer Herzfrequenzfluktuation in Bezug auf die Zeit, die bei dem Erfassen erfasst wird, wobei das Merkmal einen Mittelwert und eine Variation, bevor eine Zunahme der Herzfrequenz detektiert wird, und ein Mittelwert und eine Variation, nachdem die Herzfrequenz nach der Zunahme auf einen vorbestimmten Wert zurückgekehrt ist, aufweist; und

Bestimmen, ob die bei dem Erfassen erfasste Herzfrequenzfluktuation durch eine Getränkeeinnahme verursacht wird, basierend auf dem bei dem Extrahieren extrahierten Merkmal und den vorab in einem Speicher gespeicherten Informationen, die der Herzfrequenzfluktuation einer Getränkeeinnahme zugeordnet sind.

**18.** Informationsverarbeitungsprogramm, die den Computer veranlasst, das Informationsverarbeitungsverfahren nach Anspruch 17 auszuführen.

**Revendications**

**1.** Dispositif de traitement d'informations (100) comprenant :

une unité d'acquisition de fréquence cardiaque (10) configurée pour acquérir une fréquence cardiaque d'un objet ;

une unité d'extraction de caractéristique (20) configurée pour extraire une caractéristique à partir d'une fluctuation de fréquence cardiaque en fonction du temps qui est acquise par l'unité d'acquisition de fréquence cardiaque (10), la caractéristique comprenant une moyenne et une variation avant qu'une augmentation de la fréquence cardiaque ne soit détectée et une moyenne et une variation après un retour de la fréquence cardiaque à une valeur prédéterminée après l'augmentation ;

une mémoire (40) configurée pour, à l'avance, stocker des informations associées à une fluctuation de fréquence cardiaque d'une consommation de boisson ; et

une unité de détermination (30) configurée pour déterminer si la fluctuation de fréquence cardiaque acquise par l'unité d'acquisition de fréquence cardiaque (10) est provoquée par une consommation de boisson, sur la base de la caractéristique extraite par l'unité d'extraction de caractéristique et des informations stockées dans la mémoire.

**2.** Dispositif de traitement d'informations (100) selon la revendication 1, **caractérisé en ce que**
l'unité de détermination (30) est configurée pour déterminer que la fluctuation de fréquence cardiaque acquise par l'unité d'acquisition de fréquence cardiaque (10) est provoquée par la consommation de boisson, lorsqu'une coïncidence entre la caractéristique extraite par l'unité d'extraction de caractéristique et une caractéristique extraite des informations stockées dans la mémoire est égale à un seuil ou plus.

**3.** Dispositif de traitement d'informations (100) selon la revendication 1, **caractérisé en ce que** :

la mémoire (40) est configurée pour, à l'avance, stocker des informations associées à une fluctuation de fréquence cardiaque en fonction du temps d'une non-consommation de boisson ; et

l'unité de détermination (30) est configurée pour déterminer que la fluctuation de fréquence cardiaque acquise par l'unité d'acquisition de fréquence cardiaque (10) est provoquée par la consommation de boisson, lorsqu'une coïncidence entre la caractéristique extraite par l'unité d'extraction de caractéristique et une caractéristique extraite des informations stockées dans la mémoire est supérieure à une coïncidence entre la caractéristique extraite par l'unité d'extraction de caractéristique et une caractéristique de fréquence cardiaque de la non-consommation de boisson extraite des informations stockées dans la mémoire.

**4.** Dispositif de traitement d'informations (100) selon l'une quelconque des revendications 1 à 3, **caractérisé en ce que**
la coïncidence est calculée sur une base de l'une parmi une distance d'Euclide, une distance d'Euclide normalisée et une distance de Mahalanobis.

**5.** Dispositif de traitement d'informations (100) selon l'une quelconque des revendications 1 à 4, **caractérisé en ce qu'**il comprend en outre :

un dispositif de mesure d'informations d'action (106) configuré pour mesurer des informations d'action de l'objet, dans lequel l'unité de détermination (30) est configurée pour utiliser les informations d'action mesurées par le dispositif de mesure d'informations d'action lorsqu'il est déterminé si la fluctuation de fréquence cardiaque acquise par l'unité d'acquisition de fréquence cardiaque (10) est provoquée par la consommation de boisson.

6. Dispositif de traitement d'informations (100) selon l'une quelconque des revendications 1 à 5, **caractérisé en ce qu'**il comprend en outre :

une unité de quantification (50) configurée pour quantifier une fonction corporelle de l'objet sur une base d'une relation entre la caractéristique extraite par l'unité d'extraction de caractéristique (20) et les informations stockées dans la mémoire (40) lorsque l'unité de détermination (30) détermine que la fluctuation de fréquence cardiaque acquise par l'unité d'acquisition de fréquence cardiaque (10) est provoquée par la consommation de boisson.

7. Dispositif de traitement d'informations (100) selon l'une quelconque des revendications 1 à 6, **caractérisé en ce qu'**il comprend en outre :

une unité d'estimation de quantité de consommation de boisson (30) configurée pour estimer une quantité de consommation de boisson en utilisant la caractéristique extraite par l'unité d'extraction de caractéristique et une période pour une ingestion d'une boisson qui est stockée à l'avance dans la mémoire, lorsque l'unité de détermination détermine que la fluctuation de fréquence cardiaque acquise par l'unité d'acquisition de fréquence cardiaque (10) est provoquée par la consommation de boisson.

8. Dispositif de traitement d'informations (100) selon l'une quelconque des revendications 1 à 6, **caractérisé en ce que** la caractéristique comprend au moins l'une parmi une vitesse croissante de fréquences cardiaques et une vitesse décroissante de fréquences cardiaques.

9. Dispositif de traitement d'informations (100) selon l'une quelconque des revendications 1 à 6, **caractérisé en ce que** la caractéristique comprend une erreur entre une fluctuation de fréquence cardiaque en fonction du temps et une ligne approchée par approximation de la fluctuation de fréquence cardiaque.

10. Dispositif de traitement d'informations (100) selon l'une quelconque des revendications 1 à 6, **caractérisé en ce que** la caractéristique comprend un angle d'intersection entre une ligne approchée par approximation de fluctuation de fréquence cardiaque avant un temps prédéterminé et une autre ligne approchée par approximation de la fluctuation de fréquence cardiaque après un temps d'une fréquence cardiaque maximale.

11. Dispositif de traitement d'informations (100) selon la revendication 10, **caractérisé en ce que** le temps prédéterminé est le temps de la fréquence cardiaque maximale.

12. Dispositif de traitement d'informations (100) selon l'une quelconque des revendications 1 à 6, **caractérisé en ce que** la caractéristique comprend une valeur basée sur une valeur de crête de la fréquence cardiaque.

13. Dispositif de traitement d'informations (100) selon l'une quelconque des revendications 1 à 6, dans lequel la caractéristique comprend une valeur intégrale allant d'une augmentation de la fréquence cardiaque jusqu'à un retour à une valeur prédéterminée après l'augmentation.

14. Dispositif de traitement d'informations (100) selon l'une quelconque des revendications 1 à 6, **caractérisé en ce que** la caractéristique comprend un motif d'onde de la fluctuation de fréquence cardiaque.

15. Dispositif de traitement d'informations (100) selon l'une quelconque des revendications 1 à 6, **caractérisé en ce que** la caractéristique comprend un ou plusieurs facteurs parmi une vitesse croissante de la fréquence cardiaque, une vitesse décroissante de la fréquence cardiaque, une erreur entre une fluctuation de fréquence cardiaque et une ligne approchée par approximation de la fluctuation de fréquence cardiaque, un angle d'intersection entre une ligne approchée par approximation de la fluctuation de fréquence cardiaque avant un temps de la fréquence cardiaque maximale et une autre ligne approchée par approximation de la fluctuation de fréquence cardiaque après le temps de la fréquence cardiaque maximale, un valeur basée sur une valeur de crête de la fréquence cardiaque, une valeur intégrale allant de l'augmentation de la fréquence cardiaque jusqu'à un retour à une valeur prédéterminée après l'augmentation, et un motif d'onde de la fluctuation de fréquence cardiaque.

16. Dispositif de traitement d'informations (100) selon l'une quelconque des revendications 1 à 6, **caractérisé en ce que** la caractéristique comprend une vitesse croissante de la fréquence cardiaque, une vitesse décroissante de la fréquence cardiaque, une erreur entre la fluctuation de fréquence cardiaque et une ligne approchée par approximation de la fluctuation de fréquence cardiaque, et un angle d'intersection entre une ligne approchée par approximation de la fluctuation de fréquence cardiaque avant un temps d'une fréquence cardiaque maximale et une autre ligne approchée par approximation de la fluctuation de fréquence cardiaque après le temps de la fréquence cardiaque maximale.

17. Procédé de traitement d'informations exécuté par un ordinateur, le procédé de traitement d'informations comprenant les étapes consistant à :

acquérir une fréquence cardiaque d'un objet ;
extraire une caractéristique à partir d'une fluctuation de fréquence cardiaque en fonction du temps qui est acquise lors de l'acquisition, la caractéristique comprenant une moyenne et une variation avant qu'une augmentation de la fréquence cardiaque ne soit détectée et une moyenne et une variation après un retour de la fréquence cardiaque a une valeur prédéterminée après l'augmentation ; et
déterminer si la fluctuation de fréquence cardiaque acquise lors de l'acquisition est provoquée par une consommation de boisson, sur une base de la caractéristique extraite lors de l'extraction et d'informations stockées, à l'avance, dans une mémoire stockant les informations associées à une fluctuation de fréquence cardiaque d'une consommation de boisson.

18. Programme de traitement d'informations qui amène l'ordinateur à mettre en œuvre le procédé de traitement d'informations selon la revendication 17.

FIG. 1

FIG. 2A

40
STORAGE

10
HEART RATE ACQUISITION UNIT

20
HEARTBEAT FLUCTUATION CALCULATION UNIT

30
DETERMINATION UNIT

FIG. 2B

20

21
SPEED FEATURE CALCULATION UNIT

22
DIFFERENCE FEATURE CALCULATION UNIT

23
INTERSECTION FEATURE CALCULATION UNIT

24
BASELINE FEATURE CALCULATION UNIT

25
FLUCTUATION FEATURE CALCULATION UNIT

26
AMPLITUDE FEATURE CALCULATION UNIT

27
AREA FEATURE CALCULATION UNIT

## FIG. 3

```
        ( START )
           │
           ▼
┌──────────────────────┐
│    ACQUIRE HEART     │ ── S1
│   RATE FLUCTUATION   │
└──────────────────────┘
           │
           ▼
┌──────────────────────┐
│ CALCULATE FEATURE    │
│ OF DRINK INTAKE      │ ── S2
│ FROM HEART RATE      │
│ FLUCTUATION          │
└──────────────────────┘
           │
           ▼
┌──────────────────────┐
│   DETERMINE DRINK    │ ── S3
│    INTAKE OR NOT     │
└──────────────────────┘
           │
           ▼
      ◇ S4
  No  MEASURING IS
◄──── FINISHED?
           │ Yes
           ▼
       ( END )
```

FIG. 4

FIG. 5

```
        ┌─────────┐
        │  START  │
        └─────────┘
             │
             ▼
┌──────────────────────────┐
│  CALCULATE SPEED FEATURE │ ───S11
└──────────────────────────┘
             │
             ▼
┌──────────────────────────┐
│        CALCULATE         │ ───S12
│    DIFFERENCE FEATURE    │
└──────────────────────────┘
             │
             ▼
┌──────────────────────────┐
│        CALCULATE         │ ───S13
│   INTERSECTION FEATURE   │
└──────────────────────────┘
             │
             ▼
┌──────────────────────────┐
│        CALCULATE         │ ───S14
│     BASELINE FEATURE     │
└──────────────────────────┘
             │
             ▼
┌──────────────────────────┐
│        CACLUALTE         │ ───S15
│   FLUCTUATION FEATURE    │
└──────────────────────────┘
             │
             ▼
┌──────────────────────────┐
│        CACLUALTE         │ ───S16
│    AMPLITUDE FEATURE     │
└──────────────────────────┘
             │
             ▼
┌──────────────────────────┐
│     CALCULATE AREA       │ ───S17
│        FEATURE           │
└──────────────────────────┘
             │
             ▼
        ┌─────────┐
        │   END   │
        └─────────┘
```

FIG. 6

APPROXIMA-
TED LINE
$y_u = \alpha_u + b_u$

APPROXIMA-
TED LINE
$y_d = \alpha_d + b_d$

HEART RATE

TIME

CANDIDATE OF START
TIME OF DRINK INTAKE

TIME OF MAXIMUM
HEART RATE

TIME OF MINIMUM
HEART RATE

EP 3 372 154 B1

## FIG. 7A
### <CALCULATION OF SPEED FEATURE>

START

↓

CALCULATE START TIME CANDIDATE OF INTAKE — S21

↓

CALCULATE TIME OF MAXIMUM HEART RATE — S22

↓

CALCULATE INCREASING SPEED OF HEART RATE BY FUNCTION APPROXIMATION — S23

↓

CALCULATE TIME OF MINIMUM HEART RATE — S24

↓

CALCULATE DECREASING SPEED OF HEART RATE BY FUNCTION APPROXIMATION — S25

↓

CALCULATE RATIO OF INCREASING AND DECREASING SPEED OF HEART RATE FROM RATIO OF INCREASING SPEED AND DECREASING SPEED — S26

↓

END

## FIG. 7B
### <CALCULATION OF DIFFERENCE FEATURE>

START

↓

DETERMINE INCREASING PERIOD — S31

↓

CALCULATE DIFFERENCE OF INCREASING PERIOD BY DIFFERENCE OF MEASURED VALUE AND APPROXIMATED LINE — S32

↓

DETERMINE DECREASING PERIOD — S33

↓

CALCULATE DIFFERENCE OF DECREASING PERIOD BY DIFFERENCE OF MEASURED VALUE AND APPROXIMATED LINE — S34

↓

CALCULATE DIFFERENCE RATIO OF DECREASING PERIOD OF HEART RATE FROM RATIO OF DIFFERENCES OF INCREASING AND DECREASING PERIODS — S35

↓

END

## FIG. 7C
### <CALCULATION OF INTERSECTION FEATURE>

START

↓

CALCULATE INTERSECTION ANGLE FROM ANGLE BETWEEN APPROXIMATED LINES OF INCREASING PERIOD AND DECREASING PERIOD — S41

↓

END

FIG. 8

INCREASING PERIOD

DECREASING PERIOD

HEART RATE

$y_u$

$\hat{y}_u$

$\bar{y}$

$\hat{y}_d$

$\bar{y}$

$y_d$

CANDIDATE OF START
TIME OF DRINK INTAKE

TIME OF MAXIMUM
HEART RATE

TIME OF MINIMUM
HEART RATE

TIME

EP 3 372 154 B1

FIG. 9

INCREASING PERIOD | DECREASING PERIOD

HEART RATE

APPROXIMA-
TED LINE
$y_u = \alpha_u + b_u$

$\eta$

$\xi$

APPROXIMA-
TED LINE
$y_d = \alpha_d + b_d$

INTERSECTION
ANGLE

$\theta$

TIME OF MAXIMUM
HEART RATE

TIME

EP 3 372 154 B1

FIG. 10

PERIOD BEFORE
INCREASING

PERIOD AFTER
DECREASING

HEART RATE

AVERAGE $\mu_u$ OF HEART RATE OF
PERIOD BEFORE INCREASING

AVERAGE $\mu_d$ OF HEART
RATE OF PERIOD AFTER
DECREASING

CANDIDATE OF START
TIME OF DRINK INTAKE

TIME OF MINIMUM
HEART RATE

TIME

EP 3 372 154 B1

## FIG. 11A
### \<CALCULATION OF BASELINE FEATURE\>

```
START
  ↓
DETERMINE PERIOD BEFORE INCREASING ─ 51
  ↓
CALCULATE BASELINE OF PERIOD BEFORE INCREASING FROM AVERAGE HEART RATE OF THE PERIOD ─ S52
  ↓
DETERMINE PERIOD AFTER DECREASING ─ S53
  ↓
CALCULATE BASELINE OF PERIOD AFTER DECREASING FROM AVERAGE HEART RATE OF THE PERIOD ─ S54
  ↓
CALCULATE BASELINE RATIO OF BEFORE INCREASING AND AFTER DECREASING FROM RATIO OF BASELINES OF BEFORE INCREASING AND AFTER DECREASING ─ S55
  ↓
END
```

## FIG. 11B
### \<CALCULATION OF FLUCTUATION FEATURE\>

```
START
  ↓
CALCULATE FLUCTUATION BEFORE INCREASING FROM DISPERSION OF HEART RATE BEFORE INCREASING ─ S61
  ↓
CALCULATE FLUCTUATION AFTER DECREASING FROM DISPERSION OF HEART RATE AFTER DECREASING ─ S62
  ↓
CALCULATE FLUCTUATION RATIO BEFORE INCREASING AND AFTER DECREASING FROM RATIO OF FLUCTUATIONS BEFORE INCREASING AND AFTER DECREASING ─ S63
  ↓
END
```

## FIG. 11C
### \<CALCULATION OF AMPLITUDE FEATURE\>

```
START
  ↓
CALCULATE HEART RATE OF TIME OF MAXIMUM HEART RATE ─ S71
  ↓
END
```

## FIG. 11D
### \<CALCULATION OF AREA FEATURE\>

```
START
  ↓
CALCULATE TOTAL OF INCREARING WIDTH FROM BASELINE OF PERIOD BEFORE INCREASING FROM CANDIDATE TIME OF WATER INTAKE TO MINIMUM HEART RATE TIME ─ S81
  ↓
END
```

EP 3 372 154 B1

EP 3 372 154 B1

FIG. 12

PERIOD BEFORE INCREASING

PERIOD AFTER DECREASING

HEART RATE

DISPERSION AT i=u:

$(y_u - \mu_u)^2$

DISPERSION AT i=d:

$(y_d - \mu_d)^2$

$\mu_u$

$\mu_d$

TIME

FIG. 13

## FIG. 14

FIG. 15

FIG. 16

START

READ HEART RATE DATA WITH LABEL IN ORDER — S91

CALCULATE FEATURE OF INCREASING AND DECREASING OF HEART RATE FLUCTUATION — S92

STORE EXTRACTED FEATURE WITH LABEL — S93

ALL DATA ARE FINISHED? — S94
No
Yes

DB FOR MODEL MAKING

MAKE MODELS OF DRINK INTAKE AND NON-INTAKE — S95

END

MODEL DB

<EXAMPLE OF LABEL-ADDED DATA>

| LABEL | HEART RATE DATA |
|---|---|
| ⋮ | ⋮ |
| DRINK INTAKE | 59 |
| DRINK NON-INTAKE | 66 |
| DRINK INTAKE | 62 |

<EXAMPLE OF DB FOR MODEL MAKING>

| LABEL | FEATURE i | FEATURE ii | ⋯ | FEATURE xv |
|---|---|---|---|---|
| ⋮ | ⋮ | ⋮ | ⋮ | ⋮ |
| DRINK INTAKE | 1.1 | −0.6 | ⋯ | 1.2 |
| DRINK NON-INTAKE | 2.2 | −0.7 | ⋯ | 2.2 |
| DRINK INTAKE | 3.3 | −0.5 | ⋯ | 5.5 |

EP 3 372 154 B1

FIG. 17

START

MODEL DB

CALCULATE COINCIDENCE (DIVERGENCE
DEGREE) BETWEEN FEATURE CALCULATED
BY HEARTBEAT FLUCTUATION
CALCULATION UNIT AND MODELS OF
DRINK INTAKE AND NON-INTAKE — S101

DETERMINE BELONGING TO
LARGER COINCIDENCE ONE — S102

END

<EXAMPLE OF DB FOR DRINK
INTAKE DETECTION>

| LABEL | AVERAGE VECTOR | COVARIANCE MATRIX |
|---|---|---|
| DRINK INTAKE | $(2.5, -0.2, \cdots, 4.3)$ | $\begin{pmatrix} 0.8 & \cdots & -0.1 \\ \vdots & \ddots & \vdots \\ -0.1 & \cdots & 2.0 \end{pmatrix}$ |
| DRINK NON-INTAKE | $(0.9, 1.1, \cdots, 0.2)$ | $\begin{pmatrix} 0.2 & \cdots & -0.2 \\ \vdots & \ddots & \vdots \\ -0.2 & \cdots & 3.0 \end{pmatrix}$ |

EP 3 372 154 B1

FIG. 18

FIG. 19

```
                    ┌─────────┐
                    │  START  │
                    └────┬────┘
                         │
    ┌───────────────────▶│
    │                    ▼
    │         ┌──────────────────────┐
    │         │   ACQUIRE HEART      │──── S1a
    │         │  RATE FLUCTUATION    │
    │         └──────────┬───────────┘
    │                    ▼
    │       ┌────────────────────────┐
    │       │ CALCULATE FEATURE OF   │
    │       │ DRINK INTAKE FROM      │──── S2a
    │       │ HEART RATE FLUCTUATION │
    │       └───────────┬────────────┘
    │                   ▼
    │         ┌──────────────────────┐
    │         │  DETERMINE DRINK     │──── S3a
    │         │  INTAKE OR NOT       │
    │         └──────────┬───────────┘
    │                    ▼
    │            S4a ╱‾‾‾‾‾‾‾‾╲
    │      No      ╱ DRINK     ╲
    │    ┌────────◀  INTAKE?    ▶
    │    │         ╲           ╱
    │    │          ╲_____╱
    │    │             │ Yes
    │    │             ▼
    │    │   ┌──────────────────────┐
    │    │   │  CALCULATE DRINK     │──── S5a
    │    │   │  INTAKE AMOUNT       │
    │    │   └──────────┬───────────┘
    │    └────────────▶ │
    │             6a    ▼
    │      No    ╱‾‾‾‾‾‾‾‾‾‾╲
    └───────────◀ MEASURING IS ▶
                 ╲ FINISHED?  ╱
                  ╲_____╱
                       │ Yes
                       ▼
                  ┌─────────┐
                  │   END   │
                  └─────────┘
```

FIG. 20

100a

101
CPU

102
RAM

103
STORAGE DEVICE

DISPLAY DEVICE
104

HEARTBEAT
MEASURING DEVICE
105

ACTION INFORMATION
DETECTION DEVICE
106

FIG. 21

START

ACQUIRE HEART RATE — S111

CALCULATE FEATURE — S112

MODEL DB

CALCULATE MAHALANOBIS DISTANCE
BETWEEN FEATURE CALCULATED BY
HEARTBEAT FLUCTUATION — S113
CALCULATION UNIT AND MODELS OF
DRINK INTAKE AND NON-INTAKE

CALCULATE RATIO OF DISTANCE
(EX: DISTANCE FROM DRINK INTAKE — S114
DISTRIBUTION/DISTANCE FROM
DRINK NON-INTAKE DISTRIBUTION)

DETERMINE ACTION WHEN
MOTION DATA IS INCLUDED — S115
IN ACQUIRED DATA

DETERMINE TIME OR PLACE
WHEN ACQUIRED DATA INCLUDES — S116
DATA OF TIME OR PLACE

THRES-
HOLD DB

DETERMINE DRINK INTAKE WHEN
DISTANCE RATIO IS THRESHOLD OR
LESS AND DETERMINE DRINK NON- — S117
INTAKE WHEN DISTANCE RATIO IS
MORE THAN THRESHOLD

S118

No — MEASURING IS
FINISHED?

Yes

END

| ACTION IN-FORMATION | THRES-HOLD |
|---|---|
| SLEEP | 0. 01 |
| OCCURR-ENCE | 0. 01 |
| EXCRETION | 0. 5 |
| 15:00 | 2 |
| ⋮ | ⋮ |

100b

FIG. 22A

STORAGE
40

10

20

30

50

HEART RATE ACQUISITION UNIT

HEARTBEAT FLUCTUATION CALCULATION UNIT

DETERMINATION UNIT

BODY FUNCTION QUANTIFICATION UNIT

FIG. 22B

20

21
SPEED FEATURE CALCULATION UNIT

22
DIFFERENCE FEATURE CALCULATION UNIT

23
INTERSECTION FEATURE CALCULATION UNIT

24
BASELINE FEATURE CALCULATION UNIT

25
FLUCTUATION FEATURE CALCULATION UNIT

26
AMPLITUDE FEATURE CALCULATION UNIT

27
AREA FEATURE CALCULATION UNIT

FIG. 23

```
        START
          │
          ▼
┌──────────────────────┐
│  ACQUIRE HEART RATE  │─── S121
└──────────────────────┘
          │
          ▼
┌──────────────────────┐
│  CALCULATE FEATURE   │─── S122
└──────────────────────┘
          │
          ▼
┌──────────────────────┐
│   DETERMINE DRINK    │─── S123
│    INTAKE OR NOT     │
└──────────────────────┘
          │                          S124
          ▼
 No   ◇ DRINK INTAKE? ◇
◄─────
          │ Yes                    ┌──────────┐
          │────────────────────────│ MODEL DB │
          ▼                        └──────────┘
┌──────────────────────┐
│ EXTRACT FEATURE OF MODEL │─── S125
└──────────────────────┘
          │
          ▼
┌──────────────────────────┐
│ CALCULATE RATIO OF FEATURE │─── S126
│ (EX: CURRENT VALUE/DB VALUE) │
└──────────────────────────┘
          │
          ▼
┌──────────────────────┐
│  QUANTIFY THIRST BASED │─── S127
│   ON INCREASING SPEED  │
└──────────────────────┘
          │
          ▼
┌──────────────────────┐
│  QUANTIFY ESOPHAGUS  │─── S128
│    MOTION BASED ON   │
│   DECREASING SPEED   │
└──────────────────────┘
          │
          ▼
 No   ◇ MEASURING IS ◇ ─── S129
◄───── │  FINISHED?
          │ Yes
          ▼
         END
```

FIG. 24A

| s(1) | s(2) | ... | s(N) |
|------|------|-----|------|
| 50   | 58   |     | 62   |

FIG. 24B

NEWLY INPUT SIGNAL

| x(1) | x(2) | ... | x(N) |
|------|------|-----|------|
| 52   | 53   |     | 68   |

FIG. 25A

FIG. 25B

**REFERENCES CITED IN THE DESCRIPTION**

**Patent documents cited in the description**

- JP 2014079506 A
- JP 2000245713 A
- US 2008300641 A1